# EUROPEAN PATENT APPLICATION

(11) **EP 3 564 251 A1**
(43) Date of publication of application: **06.11.2019**
(21) Application number: 17888291.6
(22) Date of filing: 26.12.2017
(51) Int. Cl.: C07H 15/256, A01H 5/00, A23L 27/00, A23L 27/20, C12P 19/00, C12P 21/02, C07K 14/415, C12N 15/09

(54) **NOVEL STEVIOL GLYCOSIDE, METHOD FOR PRODUCING SAME, AND SWEETENER COMPOSITION CONTAINING SAME**

(30) Priority: 27.12.2016 JP 2016253248
(71) Applicant: Suntory Holdings Limited, Osaka-shi, Osaka 530-8203 (JP)
(72) Inventor: IWAKI Kazunari, Soraku-gun Kyoto 619-0284 (JP); MIYAGAWA Katsuro, Soraku-gun Kyoto 619-0284 (JP); ONO Eiichiro, Soraku-gun Kyoto 619-0284 (JP); HIRAI Tadayoshi, Soraku-gun Kyoto 619-0284 (JP); OCHIAI Misa, Soraku-gun Kyoto 619-0284 (JP); NAGAO Koji, Kawasaki-shi Kanagawa 211-0067 (JP); URAI Soichiro, Kawasaki-shi Kanagawa 211-0067 (JP); WATANABE Takehiro, Soraku-gun Kyoto 619-0284 (JP); FUJIKAWA Kohki, Soraku-gun Kyoto 619-0284 (JP)
(74) Representative: Mewburn Ellis LLP
(86) International application number: PCT/JP2017/046806
(87) International publication number: WO 2018/124143

(57) **Abstract**

The purpose of the present invention is to determine the structure of a novel steviol glycoside that is detected in cultivars containing an abundance of Reb.C (also called dulcoside B) and that affects the quality of taste in a small amount, and to identify the quality of taste properties. According to the present invention, a compound represented by formula (1), or a derivative, salt, or hydrate thereof is provided.

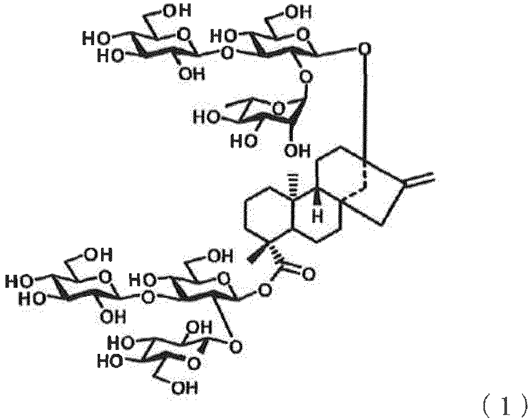

SEQUENCE LISTING

<110>  SUNTORY HOLDINGS LIMITED

<120>  Novel steviol glycoside and production method for same, and
       sweetener composition containing same

<130>  G1717WO

<150>  JP2016-253248
<151>  2016-12-27

<160>  36

<170>  PatentIn version 3.5

<210>  1
<211>  1446
<212>  DNA
<213>  Stevia rebaudiana

<220>
<221>  CDS
<222>  (1)..(1446)

<400>  1
atg gat gca atg gct aca act gag aag aaa cca cac gtc atc ttc ata      48
Met Asp Ala Met Ala Thr Thr Glu Lys Lys Pro His Val Ile Phe Ile
1               5                   10                  15

cca ttt cca gca caa agc cac att aaa gcc atg ctc aaa cta gca caa      96
Pro Phe Pro Ala Gln Ser His Ile Lys Ala Met Leu Lys Leu Ala Gln
            20                  25                  30

ctt ctc cac cac aaa gga ctc cag ata acc ttc gtc aac acc gac ttc      144
Leu Leu His His Lys Gly Leu Gln Ile Thr Phe Val Asn Thr Asp Phe
        35                  40                  45

atc cac aac cag ttt ctt gaa tca tcg ggc cca cat tgt ttg gac ggt      192
Ile His Asn Gln Phe Leu Glu Ser Ser Gly Pro His Cys Leu Asp Gly
    50                  55                  60

tca ccg ggt ttc cgg ttc gaa acc atc ccg gat ggt gtt tct cac agt      240
Ser Pro Gly Phe Arg Phe Glu Thr Ile Pro Asp Gly Val Ser His Ser
65                  70                  75                  80

ccg gaa gcg agc atc cca atc aga gaa tca ctc ttg aga tcc att gaa      288
Pro Glu Ala Ser Ile Pro Ile Arg Glu Ser Leu Leu Arg Ser Ile Glu
                85                  90                  95

acc aac ttc ttg gat cgt ttc att gat ctt gta acc aaa ctt ccg gat      336
Thr Asn Phe Leu Asp Arg Phe Ile Asp Leu Val Thr Lys Leu Pro Asp
            100                 105                 110

cct ccg act tgt att atc tca gat ggg ttc ttg tcg gtt ttc aca att      384
Pro Pro Thr Cys Ile Ile Ser Asp Gly Phe Leu Ser Val Phe Thr Ile
        115                 120                 125

gac gct gca aaa aag ctt gga att ccg gtc atg atg tat tgg aca ctt      432
Asp Ala Ala Lys Lys Leu Gly Ile Pro Val Met Met Tyr Trp Thr Leu
    130                 135                 140

gct gcc tgt ggg ttc atg ggt ttt tac cat att cat tct ctc att gag 480
Ala Ala Cys Gly Phe Met Gly Phe Tyr His Ile His Ser Leu Ile Glu
145                 150                 155                 160

aaa gga ttt gca cca ctt aaa gat gca agt tac ttg aca aat ggg tat 528
Lys Gly Phe Ala Pro Leu Lys Asp Ala Ser Tyr Leu Thr Asn Gly Tyr
                165                 170                 175

ttg gac acc gtc att gat tgg gtt ccg gga atg gaa ggc atc cgt ctc 576
Leu Asp Thr Val Ile Asp Trp Val Pro Gly Met Glu Gly Ile Arg Leu
            180                 185                 190

aag gat ttc ccg ctg gac tgg agc act gac ctc aat gac aaa gtt ttg 624
Lys Asp Phe Pro Leu Asp Trp Ser Thr Asp Leu Asn Asp Lys Val Leu
        195                 200                 205

atg ttc act aca gaa gct cct caa agg tca cac aag gtt tca cat cat 672
Met Phe Thr Thr Glu Ala Pro Gln Arg Ser His Lys Val Ser His His
    210                 215                 220

att ttc cac acg ttc gat gag ttg gag cct agt att ata aaa act ttg 720
Ile Phe His Thr Phe Asp Glu Leu Glu Pro Ser Ile Ile Lys Thr Leu
225                 230                 235                 240

tca ttg agg tat aat cac att tac acc atc ggc cca ctg caa tta ctt 768
Ser Leu Arg Tyr Asn His Ile Tyr Thr Ile Gly Pro Leu Gln Leu Leu
                245                 250                 255

ctt gat caa ata ccc gaa gag aaa aag caa act gga att acg agt ctc 816
Leu Asp Gln Ile Pro Glu Glu Lys Lys Gln Thr Gly Ile Thr Ser Leu
            260                 265                 270

cat gga tac agt tta gta aaa gaa gaa cca gag tgt ttc cag tgg ctt 864
His Gly Tyr Ser Leu Val Lys Glu Glu Pro Glu Cys Phe Gln Trp Leu
        275                 280                 285

cag tct aaa gaa cca aat tcc gtc gtt tat gta aat ttt gga agt act 912
Gln Ser Lys Glu Pro Asn Ser Val Val Tyr Val Asn Phe Gly Ser Thr
    290                 295                 300

aca gta atg tct tta gaa gac atg acg gaa ttt ggt tgg gga ctt gct 960
Thr Val Met Ser Leu Glu Asp Met Thr Glu Phe Gly Trp Gly Leu Ala
305                 310                 315                 320

aat agc aac cat tat ttc ctt tgg atc atc cga tca aac ttg gtg ata 1008
Asn Ser Asn His Tyr Phe Leu Trp Ile Ile Arg Ser Asn Leu Val Ile
                325                 330                 335

ggg gaa aat gca gtt ttg ccc cct gaa ctt gag gaa cat ata aag aaa 1056
Gly Glu Asn Ala Val Leu Pro Pro Glu Leu Glu Glu His Ile Lys Lys
            340                 345                 350

aga ggc ttt att gct agc tgg tgt tca caa gaa aag gtc ttg aag cac 1104
Arg Gly Phe Ile Ala Ser Trp Cys Ser Gln Glu Lys Val Leu Lys His
        355                 360                 365

cct tcg gtt gga ggg ttc ttg act cat tgt ggg tgg gga tcg acc atc 1152
Pro Ser Val Gly Gly Phe Leu Thr His Cys Gly Trp Gly Ser Thr Ile
    370                 375                 380

gag agc ttg tct gct ggg gtg cca atg ata tgc tgg cct tat tcg tgg 1200
Glu Ser Leu Ser Ala Gly Val Pro Met Ile Cys Trp Pro Tyr Ser Trp
385                 390                 395                 400

gac cag ctg acc aac tgt agg tat ata tgc aaa gaa tgg gag gtt ggg 1248
Asp Gln Leu Thr Asn Cys Arg Tyr Ile Cys Lys Glu Trp Glu Val Gly
                405                 410                 415

ctc gag atg gga acc aaa gtg aaa cga gat gaa gtc aag agg ctt gta 1296
Leu Glu Met Gly Thr Lys Val Lys Arg Asp Glu Val Lys Arg Leu Val
            420                 425                 430

caa gag ttg atg gga gaa gga ggt cac aaa atg agg aac aag gct aaa 1344
Gln Glu Leu Met Gly Glu Gly Gly His Lys Met Arg Asn Lys Ala Lys
        435                 440                 445

gat tgg aaa gaa aag gct cgc att gca ata gct cct aac ggt tca tct 1392
Asp Trp Lys Glu Lys Ala Arg Ile Ala Ile Ala Pro Asn Gly Ser Ser
    450                 455                 460

tct ttg aac ata gac aaa atg gtc aag gaa atc acc gtg cta gca aga 1440
Ser Leu Asn Ile Asp Lys Met Val Lys Glu Ile Thr Val Leu Ala Arg
465                 470                 475                 480

aac tag 1446
Asn

<210> 2
<211> 481
<212> PRT
<213> Stevia rebaudiana

<400> 2

Met Asp Ala Met Ala Thr Thr Glu Lys Lys Pro His Val Ile Phe Ile
1               5                   10                  15

Pro Phe Pro Ala Gln Ser His Ile Lys Ala Met Leu Lys Leu Ala Gln
            20                  25                  30

Leu Leu His His Lys Gly Leu Gln Ile Thr Phe Val Asn Thr Asp Phe
        35                  40                  45

Ile His Asn Gln Phe Leu Glu Ser Ser Gly Pro His Cys Leu Asp Gly
    50                  55                  60

Ser Pro Gly Phe Arg Phe Glu Thr Ile Pro Asp Gly Val Ser His Ser
65                  70                  75                  80

Pro Glu Ala Ser Ile Pro Ile Arg Glu Ser Leu Leu Arg Ser Ile Glu
                85                  90                  95

Thr Asn Phe Leu Asp Arg Phe Ile Asp Leu Val Thr Lys Leu Pro Asp
            100                 105                 110

Pro Pro Thr Cys Ile Ile Ser Asp Gly Phe Leu Ser Val Phe Thr Ile
        115                 120                 125

Asp Ala Ala Lys Lys Leu Gly Ile Pro Val Met Met Tyr Trp Thr Leu
    130                 135                 140

Ala Ala Cys Gly Phe Met Gly Phe Tyr His Ile His Ser Leu Ile Glu
145                 150                 155                 160

Lys Gly Phe Ala Pro Leu Lys Asp Ala Ser Tyr Leu Thr Asn Gly Tyr
                165                 170                 175

Leu Asp Thr Val Ile Asp Trp Val Pro Gly Met Glu Gly Ile Arg Leu
            180                 185                 190

Lys Asp Phe Pro Leu Asp Trp Ser Thr Asp Leu Asn Asp Lys Val Leu
        195                 200                 205

Met Phe Thr Thr Glu Ala Pro Gln Arg Ser His Lys Val Ser His His
    210                 215                 220

Ile Phe His Thr Phe Asp Glu Leu Glu Pro Ser Ile Ile Lys Thr Leu
225                 230                 235                 240

Ser Leu Arg Tyr Asn His Ile Tyr Thr Ile Gly Pro Leu Gln Leu Leu
                245                 250                 255

Leu Asp Gln Ile Pro Glu Glu Lys Lys Gln Thr Gly Ile Thr Ser Leu
            260                 265                 270

His Gly Tyr Ser Leu Val Lys Glu Glu Pro Glu Cys Phe Gln Trp Leu
        275                 280                 285

Gln Ser Lys Glu Pro Asn Ser Val Val Tyr Val Asn Phe Gly Ser Thr
    290                 295                 300

Thr Val Met Ser Leu Glu Asp Met Thr Glu Phe Gly Trp Gly Leu Ala
305                 310                 315                 320

Asn Ser Asn His Tyr Phe Leu Trp Ile Ile Arg Ser Asn Leu Val Ile
                325                 330                 335

Gly Glu Asn Ala Val Leu Pro Pro Glu Leu Glu Glu His Ile Lys Lys
            340                 345                 350

Arg Gly Phe Ile Ala Ser Trp Cys Ser Gln Glu Lys Val Leu Lys His
        355                 360                 365

Pro Ser Val Gly Gly Phe Leu Thr His Cys Gly Trp Gly Ser Thr Ile

370                 375                 380

Glu Ser Leu Ser Ala Gly Val Pro Met Ile Cys Trp Pro Tyr Ser Trp
385                 390                 395                 400

Asp Gln Leu Thr Asn Cys Arg Tyr Ile Cys Lys Glu Trp Glu Val Gly
                405                 410                 415

Leu Glu Met Gly Thr Lys Val Lys Arg Asp Glu Val Lys Arg Leu Val
            420                 425                 430

Gln Glu Leu Met Gly Glu Gly Gly His Lys Met Arg Asn Lys Ala Lys
        435                 440                 445

Asp Trp Lys Glu Lys Ala Arg Ile Ala Ile Ala Pro Asn Gly Ser Ser
    450                 455                 460

Ser Leu Asn Ile Asp Lys Met Val Lys Glu Ile Thr Val Leu Ala Arg
465                 470                 475                 480

Asn

<210>  3
<211>  1383
<212>  DNA
<213>  Stevia rebaudiana

<220>
<221>  CDS
<222>  (1)..(1383)

<400>  3
atg gcg gaa caa caa aag atc aag aaa tca cca cac gtt cta ctc atc        48
Met Ala Glu Gln Gln Lys Ile Lys Lys Ser Pro His Val Leu Leu Ile
1               5                   10                  15

cca ttc cct tta caa ggc cat ata aac cct ttc atc cag ttt ggc aaa        96
Pro Phe Pro Leu Gln Gly His Ile Asn Pro Phe Ile Gln Phe Gly Lys
            20                  25                  30

cga tta atc tcc aaa ggt gtc aaa aca aca ctt gtt acc acc atc cac       144
Arg Leu Ile Ser Lys Gly Val Lys Thr Thr Leu Val Thr Thr Ile His
        35                  40                  45

acc tta aac tca acc cta aac cac agt aac acc acc acc acc tcc atc       192
Thr Leu Asn Ser Thr Leu Asn His Ser Asn Thr Thr Thr Thr Ser Ile
    50                  55                  60

gaa atc caa gca att tcc gat ggt tgt gat gaa ggc ggt ttt atg agt       240
Glu Ile Gln Ala Ile Ser Asp Gly Cys Asp Glu Gly Gly Phe Met Ser
65                  70                  75                  80

gca gga gaa tca tat ttg gaa aca ttc aaa caa gtt ggg tct aaa tca       288

Ala Gly Glu Ser Tyr Leu Glu Thr Phe Lys Gln Val Gly Ser Lys Ser
                85                  90                  95

cta gct gac tta atc aag aag ctt caa agt gaa gga acc aca att gat      336
Leu Ala Asp Leu Ile Lys Lys Leu Gln Ser Glu Gly Thr Thr Ile Asp
            100                 105                 110

gca atc att tat gat tct atg act gaa tgg gtt tta gat gtt gca att      384
Ala Ile Ile Tyr Asp Ser Met Thr Glu Trp Val Leu Asp Val Ala Ile
        115                 120                 125

gag ttt gga atc gat ggt ggt tcg ttt ttc act caa gct tgt gtt gta      432
Glu Phe Gly Ile Asp Gly Gly Ser Phe Phe Thr Gln Ala Cys Val Val
    130                 135                 140

aac agc tta tat tat cat gtt cat aag ggt ttg att tct ttg cca ttg      480
Asn Ser Leu Tyr Tyr His Val His Lys Gly Leu Ile Ser Leu Pro Leu
145                 150                 155                 160

ggt gaa act gtt tcg gtt cct gga ttt cca gag ctt caa cgg tgg gag      528
Gly Glu Thr Val Ser Val Pro Gly Phe Pro Glu Leu Gln Arg Trp Glu
                165                 170                 175

aca ccg tta att ttg cag aat cat gag caa ata cag agc cct tgg tct      576
Thr Pro Leu Ile Leu Gln Asn His Glu Gln Ile Gln Ser Pro Trp Ser
            180                 185                 190

cag atg ttg ttt ggt cag ttt gct aat att gat caa gca cgt tgg gtc      624
Gln Met Leu Phe Gly Gln Phe Ala Asn Ile Asp Gln Ala Arg Trp Val
        195                 200                 205

ttc aca aat agt ttt tac aag ctc gag gaa gag gta ata gag tgg acg      672
Phe Thr Asn Ser Phe Tyr Lys Leu Glu Glu Glu Val Ile Glu Trp Thr
    210                 215                 220

aga aag ata tgg aac ttg aag gta atc ggg cca aca ctt cca tcc atg      720
Arg Lys Ile Trp Asn Leu Lys Val Ile Gly Pro Thr Leu Pro Ser Met
225                 230                 235                 240

tac ctt gac aaa cga ctt gat gat gat aaa gat aac gga ttt aat ctc      768
Tyr Leu Asp Lys Arg Leu Asp Asp Asp Lys Asp Asn Gly Phe Asn Leu
                245                 250                 255

tac aaa gca aac cat cat gag tgc atg aac tgg tta gac gat aag cca      816
Tyr Lys Ala Asn His His Glu Cys Met Asn Trp Leu Asp Asp Lys Pro
            260                 265                 270

aag gaa tca gtt gtt tac gta gca ttt ggt agc ctg gtg aaa cat gga      864
Lys Glu Ser Val Val Tyr Val Ala Phe Gly Ser Leu Val Lys His Gly
        275                 280                 285

ccc gaa caa gtg gaa gaa atc aca cgg gct tta ata gat agt gat gtc      912
Pro Glu Gln Val Glu Glu Ile Thr Arg Ala Leu Ile Asp Ser Asp Val
    290                 295                 300

aac ttc ttg tgg gtt atc aaa cat aaa gaa gag gga aag ctc cca gaa      960
Asn Phe Leu Trp Val Ile Lys His Lys Glu Glu Gly Lys Leu Pro Glu
305                 310                 315                 320

aat ctt tcg gaa gta ata aaa acc gga aag ggt ttg att gta gca tgg     1008
Asn Leu Ser Glu Val Ile Lys Thr Gly Lys Gly Leu Ile Val Ala Trp
                325                 330                 335

tgc aaa caa ttg gat gtg tta gca cac gaa tca gta gga tgc ttt gtt 1056
Cys Lys Gln Leu Asp Val Leu Ala His Glu Ser Val Gly Cys Phe Val
            340                 345                 350

aca cat tgt ggg ttc aac tca act ctt gaa gca ata agt ctt gga gtc 1104
Thr His Cys Gly Phe Asn Ser Thr Leu Glu Ala Ile Ser Leu Gly Val
        355                 360                 365

ccc gtt gtt gca atg cct caa ttt tcg gat caa act aca aat gcc aag 1152
Pro Val Val Ala Met Pro Gln Phe Ser Asp Gln Thr Thr Asn Ala Lys
    370                 375                 380

ctt cta gat gaa att ttg ggt gtt gga gtt aga gtt aag gct gat gag 1200
Leu Leu Asp Glu Ile Leu Gly Val Gly Val Arg Val Lys Ala Asp Glu
385                 390                 395                 400

aat ggg ata gtg aga aga gga aat ctt gcg tca tgt att aag atg att 1248
Asn Gly Ile Val Arg Arg Gly Asn Leu Ala Ser Cys Ile Lys Met Ile
                405                 410                 415

atg gag gag gaa aga gga gta ata atc cga aag aat gcg gta aaa tgg 1296
Met Glu Glu Glu Arg Gly Val Ile Ile Arg Lys Asn Ala Val Lys Trp
            420                 425                 430

aag gat ttg gct aaa gta gcc gtt cat gaa ggt ggt agc tca gac aat 1344
Lys Asp Leu Ala Lys Val Ala Val His Glu Gly Gly Ser Ser Asp Asn
        435                 440                 445

gat att gtc gaa ttt gta agt gag cta att aag gct taa 1383
Asp Ile Val Glu Phe Val Ser Glu Leu Ile Lys Ala
    450                 455                 460

<210> 4
<211> 460
<212> PRT
<213> Stevia rebaudiana

<400> 4

Met Ala Glu Gln Gln Lys Ile Lys Lys Ser Pro His Val Leu Leu Ile
1               5                   10                  15

Pro Phe Pro Leu Gln Gly His Ile Asn Pro Phe Ile Gln Phe Gly Lys
            20                  25                  30

Arg Leu Ile Ser Lys Gly Val Lys Thr Thr Leu Val Thr Thr Ile His
        35                  40                  45

Thr Leu Asn Ser Thr Leu Asn His Ser Asn Thr Thr Thr Thr Ser Ile
    50                  55                  60

Glu Ile Gln Ala Ile Ser Asp Gly Cys Asp Glu Gly Gly Phe Met Ser
65                  70                  75                  80

Ala Gly Glu Ser Tyr Leu Glu Thr Phe Lys Gln Val Gly Ser Lys Ser
                85                  90                  95

Leu Ala Asp Leu Ile Lys Lys Leu Gln Ser Glu Gly Thr Thr Ile Asp
        100                 105                 110

Ala Ile Ile Tyr Asp Ser Met Thr Glu Trp Val Leu Asp Val Ala Ile
        115                 120                 125

Glu Phe Gly Ile Asp Gly Gly Ser Phe Phe Thr Gln Ala Cys Val Val
    130                 135                 140

Asn Ser Leu Tyr Tyr His Val His Lys Gly Leu Ile Ser Leu Pro Leu
145                 150                 155                 160

Gly Glu Thr Val Ser Val Pro Gly Phe Pro Glu Leu Gln Arg Trp Glu
                165                 170                 175

Thr Pro Leu Ile Leu Gln Asn His Glu Gln Ile Gln Ser Pro Trp Ser
        180                 185                 190

Gln Met Leu Phe Gly Gln Phe Ala Asn Ile Asp Gln Ala Arg Trp Val
        195                 200                 205

Phe Thr Asn Ser Phe Tyr Lys Leu Glu Glu Glu Val Ile Glu Trp Thr
    210                 215                 220

Arg Lys Ile Trp Asn Leu Lys Val Ile Gly Pro Thr Leu Pro Ser Met
225                 230                 235                 240

Tyr Leu Asp Lys Arg Leu Asp Asp Asp Lys Asp Asn Gly Phe Asn Leu
                245                 250                 255

Tyr Lys Ala Asn His His Glu Cys Met Asn Trp Leu Asp Asp Lys Pro
        260                 265                 270

Lys Glu Ser Val Val Tyr Val Ala Phe Gly Ser Leu Val Lys His Gly
        275                 280                 285

Pro Glu Gln Val Glu Glu Ile Thr Arg Ala Leu Ile Asp Ser Asp Val
    290                 295                 300

Asn Phe Leu Trp Val Ile Lys His Lys Glu Glu Gly Lys Leu Pro Glu
305                 310                 315                 320

Asn Leu Ser Glu Val Ile Lys Thr Gly Lys Gly Leu Ile Val Ala Trp
                325                 330                 335

Cys Lys Gln Leu Asp Val Leu Ala His Glu Ser Val Gly Cys Phe Val
        340                 345                 350

Thr His Cys Gly Phe Asn Ser Thr Leu Glu Ala Ile Ser Leu Gly Val
        355                 360                 365

Pro Val Val Ala Met Pro Gln Phe Ser Asp Gln Thr Thr Asn Ala Lys
    370                 375                 380

Leu Leu Asp Glu Ile Leu Gly Val Gly Val Arg Val Lys Ala Asp Glu
385                 390                 395                 400

Asn Gly Ile Val Arg Arg Gly Asn Leu Ala Ser Cys Ile Lys Met Ile
                405                 410                 415

Met Glu Glu Glu Arg Gly Val Ile Ile Arg Lys Asn Ala Val Lys Trp
            420                 425                 430

Lys Asp Leu Ala Lys Val Ala Val His Glu Gly Gly Ser Ser Asp Asn
        435                 440                 445

Asp Ile Val Glu Phe Val Ser Glu Leu Ile Lys Ala
    450                 455                 460

<210>  5
<211>  1458
<212>  DNA
<213>  Stevia rebaudiana

<220>
<221>  CDS
<222>  (1)..(1458)

<400>  5
atg tac aac gtt act tat cat caa aat tca aaa gca atg gct acc agt      48
Met Tyr Asn Val Thr Tyr His Gln Asn Ser Lys Ala Met Ala Thr Ser
1               5                   10                  15

gac tcc ata gtt gac gac cgt aag cag ctt cat gtt gcg acg ttc cca      96
Asp Ser Ile Val Asp Asp Arg Lys Gln Leu His Val Ala Thr Phe Pro
            20                  25                  30

tgg ctt gct ttc ggt cac atc ctc cct tac ctt cag ctt tcg aaa ttg      144
Trp Leu Ala Phe Gly His Ile Leu Pro Tyr Leu Gln Leu Ser Lys Leu
        35                  40                  45

ata gct gaa aag ggt cac aaa gtc tcg ttt ctt tct acc acc aga aac      192
Ile Ala Glu Lys Gly His Lys Val Ser Phe Leu Ser Thr Thr Arg Asn
    50                  55                  60

att caa cgt ctc tct tct cat atc tcg cca ctc ata aat gtt gtt caa      240
Ile Gln Arg Leu Ser Ser His Ile Ser Pro Leu Ile Asn Val Val Gln
65                  70                  75                  80

ctc aca ctt cca cgt gtc caa gag ctg ccg gag gat gca gag gcg acc      288
Leu Thr Leu Pro Arg Val Gln Glu Leu Pro Glu Asp Ala Glu Ala Thr

                85                  90                  95

act gac gtc cac cct gaa gat att cca tat ctc aag aag gct tct gat      336
Thr Asp Val His Pro Glu Asp Ile Pro Tyr Leu Lys Lys Ala Ser Asp
            100                 105                 110

ggt ctt caa ccg gag gtc acc cgg ttt cta gaa caa cac tct ccg gac      384
Gly Leu Gln Pro Glu Val Thr Arg Phe Leu Glu Gln His Ser Pro Asp
        115                 120                 125

tgg att att tat gat tat act cac tac tgg ttg cca tcc atc gcg gct      432
Trp Ile Ile Tyr Asp Tyr Thr His Tyr Trp Leu Pro Ser Ile Ala Ala
    130                 135                 140

agc ctc ggt atc tca cga gcc cac ttc tcc gtc acc act cca tgg gcc      480
Ser Leu Gly Ile Ser Arg Ala His Phe Ser Val Thr Thr Pro Trp Ala
145                 150                 155                 160

att gct tat atg gga ccc tca gct gac gcc atg ata aat ggt tca gat      528
Ile Ala Tyr Met Gly Pro Ser Ala Asp Ala Met Ile Asn Gly Ser Asp
                165                 170                 175

ggt cga acc acg gtt gag gat ctc acg aca ccg ccc aag tgg ttt ccc      576
Gly Arg Thr Thr Val Glu Asp Leu Thr Thr Pro Pro Lys Trp Phe Pro
            180                 185                 190

ttt ccg acc aaa gta tgc tgg cgg aag cat gat ctt gcc cga ctg gtg      624
Phe Pro Thr Lys Val Cys Trp Arg Lys His Asp Leu Ala Arg Leu Val
        195                 200                 205

cct tac aaa gct ccg ggg ata tct gat gga tac cgt atg ggg ctg gtt      672
Pro Tyr Lys Ala Pro Gly Ile Ser Asp Gly Tyr Arg Met Gly Leu Val
    210                 215                 220

ctt aag gga tct gat tgt ttg ctt tcc aaa tgt tac cat gag ttt gga      720
Leu Lys Gly Ser Asp Cys Leu Leu Ser Lys Cys Tyr His Glu Phe Gly
225                 230                 235                 240

act caa tgg cta cct ctt ttg gag aca cta cac caa gta ccg gtg gtt      768
Thr Gln Trp Leu Pro Leu Leu Glu Thr Leu His Gln Val Pro Val Val
                245                 250                 255

ccg gtg gga tta ctg cca ccg gaa ata ccc gga gac gag aaa gat gaa      816
Pro Val Gly Leu Leu Pro Pro Glu Ile Pro Gly Asp Glu Lys Asp Glu
            260                 265                 270

aca tgg gtg tca atc aag aaa tgg ctc gat ggt aaa caa aaa ggc agt      864
Thr Trp Val Ser Ile Lys Lys Trp Leu Asp Gly Lys Gln Lys Gly Ser
        275                 280                 285

gtg gtg tac gtt gca tta gga agc gag gtt ttg gtg agc caa acc gag      912
Val Val Tyr Val Ala Leu Gly Ser Glu Val Leu Val Ser Gln Thr Glu
    290                 295                 300

gtt gtt gag tta gca ttg ggt ctc gag ctt tct ggg ttg cca ttt gtt      960
Val Val Glu Leu Ala Leu Gly Leu Glu Leu Ser Gly Leu Pro Phe Val
305                 310                 315                 320

tgg gct tat aga aaa cca aaa ggt ccc gcg aag tca gac tcg gtg gag     1008
Trp Ala Tyr Arg Lys Pro Lys Gly Pro Ala Lys Ser Asp Ser Val Glu
                325                 330                 335

ttg cca gac ggg ttc gtg gaa cga act cgt gac cgt ggg ttg gtc tgg     1056

Leu Pro Asp Gly Phe Val Glu Arg Thr Arg Asp Arg Gly Leu Val Trp
            340                 345                 350

acg agt tgg gca cct cag tta cga ata ctg agc cat gag tcg gtt tgt      1104
Thr Ser Trp Ala Pro Gln Leu Arg Ile Leu Ser His Glu Ser Val Cys
        355                 360                 365

ggt ttc ttg act cat tgt ggt tct gga tca att gtg gaa ggg cta atg      1152
Gly Phe Leu Thr His Cys Gly Ser Gly Ser Ile Val Glu Gly Leu Met
    370                 375                 380

ttt ggt cac cct cta atc atg cta ccg att ttt ggg gac caa cct ctg      1200
Phe Gly His Pro Leu Ile Met Leu Pro Ile Phe Gly Asp Gln Pro Leu
385                 390                 395                 400

aat gct cga tta ctg gag gac aaa cag gtg gga atc gag ata cca aga      1248
Asn Ala Arg Leu Leu Glu Asp Lys Gln Val Gly Ile Glu Ile Pro Arg
                405                 410                 415

aat gag gaa gat ggt tgc ttg acc aag gag tcg gtt gct aga tca ctg      1296
Asn Glu Glu Asp Gly Cys Leu Thr Lys Glu Ser Val Ala Arg Ser Leu
            420                 425                 430

agg tcc gtt gtt gtg gaa aaa gaa ggg gag atc tac aag gcg aac gcg      1344
Arg Ser Val Val Val Glu Lys Glu Gly Glu Ile Tyr Lys Ala Asn Ala
        435                 440                 445

agg gag ctg agt aaa atc tat aac gac act aag gtt gaa aaa gaa tat      1392
Arg Glu Leu Ser Lys Ile Tyr Asn Asp Thr Lys Val Glu Lys Glu Tyr
    450                 455                 460

gta agc caa ttc gta gac tat ttg gaa aag aat gcg cgt gcg gtt gcc      1440
Val Ser Gln Phe Val Asp Tyr Leu Glu Lys Asn Ala Arg Ala Val Ala
465                 470                 475                 480

atc gat cat gag agt taa                                              1458
Ile Asp His Glu Ser
                485

<210>  6
<211>  485
<212>  PRT
<213>  Stevia rebaudiana

<400>  6

Met Tyr Asn Val Thr Tyr His Gln Asn Ser Lys Ala Met Ala Thr Ser
1               5                   10                  15

Asp Ser Ile Val Asp Asp Arg Lys Gln Leu His Val Ala Thr Phe Pro
            20                  25                  30

Trp Leu Ala Phe Gly His Ile Leu Pro Tyr Leu Gln Leu Ser Lys Leu
        35                  40                  45

Ile Ala Glu Lys Gly His Lys Val Ser Phe Leu Ser Thr Thr Arg Asn
    50                  55                  60

Ile Gln Arg Leu Ser Ser His Ile Ser Pro Leu Ile Asn Val Val Gln
65                  70                  75                  80

Leu Thr Leu Pro Arg Val Gln Glu Leu Pro Glu Asp Ala Glu Ala Thr
                85                  90                  95

Thr Asp Val His Pro Glu Asp Ile Pro Tyr Leu Lys Lys Ala Ser Asp
            100                 105                 110

Gly Leu Gln Pro Glu Val Thr Arg Phe Leu Glu Gln His Ser Pro Asp
        115                 120                 125

Trp Ile Ile Tyr Asp Tyr Thr His Tyr Trp Leu Pro Ser Ile Ala Ala
    130                 135                 140

Ser Leu Gly Ile Ser Arg Ala His Phe Ser Val Thr Thr Pro Trp Ala
145                 150                 155                 160

Ile Ala Tyr Met Gly Pro Ser Ala Asp Ala Met Ile Asn Gly Ser Asp
                165                 170                 175

Gly Arg Thr Thr Val Glu Asp Leu Thr Thr Pro Pro Lys Trp Phe Pro
            180                 185                 190

Phe Pro Thr Lys Val Cys Trp Arg Lys His Asp Leu Ala Arg Leu Val
        195                 200                 205

Pro Tyr Lys Ala Pro Gly Ile Ser Asp Gly Tyr Arg Met Gly Leu Val
    210                 215                 220

Leu Lys Gly Ser Asp Cys Leu Leu Ser Lys Cys Tyr His Glu Phe Gly
225                 230                 235                 240

Thr Gln Trp Leu Pro Leu Leu Glu Thr Leu His Gln Val Pro Val Val
                245                 250                 255

Pro Val Gly Leu Leu Pro Pro Glu Ile Pro Gly Asp Glu Lys Asp Glu
            260                 265                 270

Thr Trp Val Ser Ile Lys Lys Trp Leu Asp Gly Lys Gln Lys Gly Ser
        275                 280                 285

Val Val Tyr Val Ala Leu Gly Ser Glu Val Leu Val Ser Gln Thr Glu
    290                 295                 300

Val Val Glu Leu Ala Leu Gly Leu Glu Leu Ser Gly Leu Pro Phe Val
305                 310                 315                 320

Trp Ala Tyr Arg Lys Pro Lys Gly Pro Ala Lys Ser Asp Ser Val Glu
                325                 330                 335

Leu Pro Asp Gly Phe Val Glu Arg Thr Arg Asp Arg Gly Leu Val Trp
            340                 345                 350

Thr Ser Trp Ala Pro Gln Leu Arg Ile Leu Ser His Glu Ser Val Cys
        355                 360                 365

Gly Phe Leu Thr His Cys Gly Ser Gly Ser Ile Val Glu Gly Leu Met
    370                 375                 380

Phe Gly His Pro Leu Ile Met Leu Pro Ile Phe Gly Asp Gln Pro Leu
385                 390                 395                 400

Asn Ala Arg Leu Leu Glu Asp Lys Gln Val Gly Ile Glu Ile Pro Arg
                405                 410                 415

Asn Glu Glu Asp Gly Cys Leu Thr Lys Glu Ser Val Ala Arg Ser Leu
            420                 425                 430

Arg Ser Val Val Val Glu Lys Glu Gly Glu Ile Tyr Lys Ala Asn Ala
        435                 440                 445

Arg Glu Leu Ser Lys Ile Tyr Asn Asp Thr Lys Val Glu Lys Glu Tyr
    450                 455                 460

Val Ser Gln Phe Val Asp Tyr Leu Glu Lys Asn Ala Arg Ala Val Ala
465                 470                 475                 480

Ile Asp His Glu Ser
                485

<210>  7
<211>  1377
<212>  DNA
<213>  Stevia rebaudiana

<220>
<221>  CDS
<222>  (1)..(1377)

<400>  7
atg gaa aat aaa acg gag acc acc gtt cgc cgg cgc cgg aga ata ata      48
Met Glu Asn Lys Thr Glu Thr Thr Val Arg Arg Arg Arg Arg Ile Ile
1               5                   10                  15

tta ttc ccg gta cca ttt caa ggc cac att aac cca att ctt cag cta      96
Leu Phe Pro Val Pro Phe Gln Gly His Ile Asn Pro Ile Leu Gln Leu
            20                  25                  30

gcc aat gtg ttg tac tct aaa gga ttc agt atc acc atc ttt cac acc     144
Ala Asn Val Leu Tyr Ser Lys Gly Phe Ser Ile Thr Ile Phe His Thr
        35                  40                  45

aac ttc aac aaa ccc aaa aca tct aat tac cct cac ttc act ttc aga     192
Asn Phe Asn Lys Pro Lys Thr Ser Asn Tyr Pro His Phe Thr Phe Arg
    50                  55                  60

ttc atc ctc gac aac gac cca caa gac gaa cgc att tcc aat cta ccg     240
Phe Ile Leu Asp Asn Asp Pro Gln Asp Glu Arg Ile Ser Asn Leu Pro
65                  70                  75                  80

act cat ggt ccg ctc gct ggt atg cgg att ccg att atc aac gaa cac     288
Thr His Gly Pro Leu Ala Gly Met Arg Ile Pro Ile Ile Asn Glu His
                85                  90                  95

gga gct gac gaa tta cga cgc gaa ctg gaa ctg ttg atg tta gct tct     336
Gly Ala Asp Glu Leu Arg Arg Glu Leu Glu Leu Leu Met Leu Ala Ser
            100                 105                 110

gaa gaa gat gaa gag gta tcg tgt tta atc acg gat gct ctt tgg tac     384
Glu Glu Asp Glu Glu Val Ser Cys Leu Ile Thr Asp Ala Leu Trp Tyr
        115                 120                 125

ttc gcg caa tct gtt gct gac agt ctt aac ctc cga ccg ctt gtt ttg     432
Phe Ala Gln Ser Val Ala Asp Ser Leu Asn Leu Arg Pro Leu Val Leu
    130                 135                 140

atg aca agc agc ttg ttt aat ttt cat gca cat gtt tca ctt cct cag     480
Met Thr Ser Ser Leu Phe Asn Phe His Ala His Val Ser Leu Pro Gln
145                 150                 155                 160

ttt gat gag ctt ggt tac ctc gat cct gat gac aaa acc cgt ttg gaa     528
Phe Asp Glu Leu Gly Tyr Leu Asp Pro Asp Asp Lys Thr Arg Leu Glu
                165                 170                 175

gaa caa gcg agt ggg ttt cct atg cta aaa gtg aaa gac atc aag tct     576
Glu Gln Ala Ser Gly Phe Pro Met Leu Lys Val Lys Asp Ile Lys Ser
            180                 185                 190

gcg tat tcg aac tgg caa ata ctc aaa gag ata tta ggg aag atg ata     624
Ala Tyr Ser Asn Trp Gln Ile Leu Lys Glu Ile Leu Gly Lys Met Ile
        195                 200                 205

aaa caa aca aaa gca tct tca gga gtc atc tgg aac tca ttt aag gaa     672
Lys Gln Thr Lys Ala Ser Ser Gly Val Ile Trp Asn Ser Phe Lys Glu
    210                 215                 220

ctc gaa gag tct gag ctc gaa act gtt atc cgt gag atc ccg gct cca     720
Leu Glu Glu Ser Glu Leu Glu Thr Val Ile Arg Glu Ile Pro Ala Pro
225                 230                 235                 240

agt ttc ttg ata cca ctc ccc aag cat ttg aca gcc tct tcc agc agc     768
Ser Phe Leu Ile Pro Leu Pro Lys His Leu Thr Ala Ser Ser Ser Ser
                245                 250                 255

tta cta gac cac gat cga acc gtt ttt caa tgg tta gac caa caa ccg     816
Leu Leu Asp His Asp Arg Thr Val Phe Gln Trp Leu Asp Gln Gln Pro
            260                 265                 270

cca agt tcg gta ctg tat gtt agt ttt ggt agt act agt gaa gtg gat     864
Pro Ser Ser Val Leu Tyr Val Ser Phe Gly Ser Thr Ser Glu Val Asp

275                 280                 285

gag aaa gat ttc ttg gaa ata gct cgt ggg ttg gtt gat agc aag cag        912
Glu Lys Asp Phe Leu Glu Ile Ala Arg Gly Leu Val Asp Ser Lys Gln
    290                 295                 300

tcg ttt tta tgg gtg gtt cga cct ggg ttt gtc aag ggt tcg acg tgg        960
Ser Phe Leu Trp Val Val Arg Pro Gly Phe Val Lys Gly Ser Thr Trp
305                 310                 315                 320

gtc gaa ccg ttg cca gat ggg ttc ttg ggt gaa aga gga cgt att gtg       1008
Val Glu Pro Leu Pro Asp Gly Phe Leu Gly Glu Arg Gly Arg Ile Val
                325                 330                 335

aaa tgg gtt cca cag caa gaa gtg cta gct cat gga gca ata ggc gca       1056
Lys Trp Val Pro Gln Gln Glu Val Leu Ala His Gly Ala Ile Gly Ala
            340                 345                 350

ttc tgg act cat agc gga tgg aac tct acg ttg gaa agc gtt tgt gaa       1104
Phe Trp Thr His Ser Gly Trp Asn Ser Thr Leu Glu Ser Val Cys Glu
        355                 360                 365

ggt gtt cct atg att ttc tcg gat ttt ggg ctc gat caa ccg ttg aat       1152
Gly Val Pro Met Ile Phe Ser Asp Phe Gly Leu Asp Gln Pro Leu Asn
    370                 375                 380

gct aga tac atg agt gat gtt ttg aag gta ggg gtg tat ttg gaa aat       1200
Ala Arg Tyr Met Ser Asp Val Leu Lys Val Gly Val Tyr Leu Glu Asn
385                 390                 395                 400

ggg tgg gaa aga gga gag ata gca aat gca ata aga aga gtt atg gtg       1248
Gly Trp Glu Arg Gly Glu Ile Ala Asn Ala Ile Arg Arg Val Met Val
                405                 410                 415

gat gaa gaa gga gaa tac att aga cag aat gca aga gtt ttg aaa caa       1296
Asp Glu Glu Gly Glu Tyr Ile Arg Gln Asn Ala Arg Val Leu Lys Gln
            420                 425                 430

aag gca gat gtt tct ttg atg aag ggt ggt tcg tct tac gaa tca tta       1344
Lys Ala Asp Val Ser Leu Met Lys Gly Gly Ser Ser Tyr Glu Ser Leu
        435                 440                 445

gag tct cta gtt tct tac att tca tcg ttg taa                           1377
Glu Ser Leu Val Ser Tyr Ile Ser Ser Leu
    450                 455

<210>  8
<211>  458
<212>  PRT
<213>  Stevia rebaudiana

<400>  8

Met Glu Asn Lys Thr Glu Thr Thr Val Arg Arg Arg Arg Arg Ile Ile
1               5                   10                  15

Leu Phe Pro Val Pro Phe Gln Gly His Ile Asn Pro Ile Leu Gln Leu
            20                  25                  30

Ala Asn Val Leu Tyr Ser Lys Gly Phe Ser Ile Thr Ile Phe His Thr

35                  40                  45

Asn Phe Asn Lys Pro Lys Thr Ser Asn Tyr Pro His Phe Thr Phe Arg
    50                  55                  60

Phe Ile Leu Asp Asn Asp Pro Gln Asp Glu Arg Ile Ser Asn Leu Pro
65                  70                  75                  80

Thr His Gly Pro Leu Ala Gly Met Arg Ile Pro Ile Ile Asn Glu His
                85                  90                  95

Gly Ala Asp Glu Leu Arg Arg Glu Leu Glu Leu Leu Met Leu Ala Ser
            100                 105                 110

Glu Glu Asp Glu Glu Val Ser Cys Leu Ile Thr Asp Ala Leu Trp Tyr
        115                 120                 125

Phe Ala Gln Ser Val Ala Asp Ser Leu Asn Leu Arg Pro Leu Val Leu
    130                 135                 140

Met Thr Ser Ser Leu Phe Asn Phe His Ala His Val Ser Leu Pro Gln
145                 150                 155                 160

Phe Asp Glu Leu Gly Tyr Leu Asp Pro Asp Asp Lys Thr Arg Leu Glu
                165                 170                 175

Glu Gln Ala Ser Gly Phe Pro Met Leu Lys Val Lys Asp Ile Lys Ser
            180                 185                 190

Ala Tyr Ser Asn Trp Gln Ile Leu Lys Glu Ile Leu Gly Lys Met Ile
        195                 200                 205

Lys Gln Thr Lys Ala Ser Ser Gly Val Ile Trp Asn Ser Phe Lys Glu
    210                 215                 220

Leu Glu Glu Ser Glu Leu Glu Thr Val Ile Arg Glu Ile Pro Ala Pro
225                 230                 235                 240

Ser Phe Leu Ile Pro Leu Pro Lys His Leu Thr Ala Ser Ser Ser Ser
                245                 250                 255

Leu Leu Asp His Asp Arg Thr Val Phe Gln Trp Leu Asp Gln Gln Pro
            260                 265                 270

Pro Ser Ser Val Leu Tyr Val Ser Phe Gly Ser Thr Ser Glu Val Asp
        275                 280                 285

Glu Lys Asp Phe Leu Glu Ile Ala Arg Gly Leu Val Asp Ser Lys Gln
    290                 295                 300

Ser Phe Leu Trp Val Val Arg Pro Gly Phe Val Lys Gly Ser Thr Trp
305                 310                 315                 320

Val Glu Pro Leu Pro Asp Gly Phe Leu Gly Glu Arg Gly Arg Ile Val
                325                 330                 335

Lys Trp Val Pro Gln Gln Glu Val Leu Ala His Gly Ala Ile Gly Ala
            340                 345                 350

Phe Trp Thr His Ser Gly Trp Asn Ser Thr Leu Glu Ser Val Cys Glu
        355                 360                 365

Gly Val Pro Met Ile Phe Ser Asp Phe Gly Leu Asp Gln Pro Leu Asn
    370                 375                 380

Ala Arg Tyr Met Ser Asp Val Leu Lys Val Gly Val Tyr Leu Glu Asn
385                 390                 395                 400

Gly Trp Glu Arg Gly Glu Ile Ala Asn Ala Ile Arg Arg Val Met Val
                405                 410                 415

Asp Glu Glu Gly Glu Tyr Ile Arg Gln Asn Ala Arg Val Leu Lys Gln
            420                 425                 430

Lys Ala Asp Val Ser Leu Met Lys Gly Gly Ser Ser Tyr Glu Ser Leu
        435                 440                 445

Glu Ser Leu Val Ser Tyr Ile Ser Ser Leu
    450                 455

<210>  9
<211>  2004
<212>  DNA
<213>  Arabidopsis thaliana

<220>
<221>  CDS
<222>  (1)..(2004)

<400>  9
atg gat gat act acg tat aag cca aag aac att ctc att act gga gct      48
Met Asp Asp Thr Thr Tyr Lys Pro Lys Asn Ile Leu Ile Thr Gly Ala
1               5                   10                  15

gct gga ttt att gct tct cat gtt gcc aac aga tta atc cgt aac tat      96
Ala Gly Phe Ile Ala Ser His Val Ala Asn Arg Leu Ile Arg Asn Tyr
            20                  25                  30

cct gat tac aag atc gtt gtt ctt gac aag ctt gat tac tgt tca gat     144
Pro Asp Tyr Lys Ile Val Val Leu Asp Lys Leu Asp Tyr Cys Ser Asp
        35                  40                  45

ctg aag aat ctt gat cct tct ttt tct tca cca aat ttc aag ttt gtc     192
Leu Lys Asn Leu Asp Pro Ser Phe Ser Ser Pro Asn Phe Lys Phe Val
    50                  55                  60

aaa gga gat atc gcg agt gat gat ctc gtt aac tac ctt ctc atc act     240
Lys Gly Asp Ile Ala Ser Asp Asp Leu Val Asn Tyr Leu Leu Ile Thr
65                  70                  75                  80

gaa aac att gat acg ata atg cat ttt gct gct caa act cat gtt gat     288
Glu Asn Ile Asp Thr Ile Met His Phe Ala Ala Gln Thr His Val Asp
                85                  90                  95

aac tct ttt ggt aat agc ttt gag ttt acc aag aac aat att tat ggt     336
Asn Ser Phe Gly Asn Ser Phe Glu Phe Thr Lys Asn Asn Ile Tyr Gly
            100                 105                 110

act cat gtt ctt ttg gaa gcc tgt aaa gtt aca gga cag atc agg agg     384
Thr His Val Leu Leu Glu Ala Cys Lys Val Thr Gly Gln Ile Arg Arg
        115                 120                 125

ttt atc cat gtg agt acc gat gaa gtc tat gga gaa acc gat gag gat     432
Phe Ile His Val Ser Thr Asp Glu Val Tyr Gly Glu Thr Asp Glu Asp
    130                 135                 140

gct gct gta gga aac cat gaa gct tct cag ctg tta ccg acg aat cct     480
Ala Ala Val Gly Asn His Glu Ala Ser Gln Leu Leu Pro Thr Asn Pro
145                 150                 155                 160

tac tct gca act aag gct ggt gct gag atg ctt gtg atg gct tat ggt     528
Tyr Ser Ala Thr Lys Ala Gly Ala Glu Met Leu Val Met Ala Tyr Gly
                165                 170                 175

aga tca tat gga ttg cct gtt att acg act cgc ggg aac aat gtt tat     576
Arg Ser Tyr Gly Leu Pro Val Ile Thr Thr Arg Gly Asn Asn Val Tyr
            180                 185                 190

ggg cct aac cag ttt cct gaa aaa atg att cct aag ttc atc ttg ttg     624
Gly Pro Asn Gln Phe Pro Glu Lys Met Ile Pro Lys Phe Ile Leu Leu
        195                 200                 205

gct atg agt ggg aag ccg ctt ccc atc cat gga gat gga tct aat gtc     672
Ala Met Ser Gly Lys Pro Leu Pro Ile His Gly Asp Gly Ser Asn Val
    210                 215                 220

cgg agt tac ttg tac tgc gaa gac gtt gct gag gct ttt gag gtt gtt     720
Arg Ser Tyr Leu Tyr Cys Glu Asp Val Ala Glu Ala Phe Glu Val Val
225                 230                 235                 240

ctt cac aaa gga gaa atc ggt cat gtc tac aat gtc ggc aca aaa aga     768
Leu His Lys Gly Glu Ile Gly His Val Tyr Asn Val Gly Thr Lys Arg
                245                 250                 255

gaa agg aga gtg atc gat gtg gct aga gac atc tgc aaa ctt ttc ggg     816
Glu Arg Arg Val Ile Asp Val Ala Arg Asp Ile Cys Lys Leu Phe Gly
            260                 265                 270

aaa gac cct gag tca agc att cag ttt gtg gag aac cgg ccc ttt aat     864
Lys Asp Pro Glu Ser Ser Ile Gln Phe Val Glu Asn Arg Pro Phe Asn
        275                 280                 285

gat caa agg tac ttc ctt gat gat cag aag ctg aag aaa ttg ggg tgg 912
Asp Gln Arg Tyr Phe Leu Asp Asp Gln Lys Leu Lys Lys Leu Gly Trp
    290                 295                 300

caa gag cga aca aat tgg gaa gat gga ttg aag aag aca atg gac tgg 960
Gln Glu Arg Thr Asn Trp Glu Asp Gly Leu Lys Lys Thr Met Asp Trp
305                 310                 315                 320

tac act cag aat cct gag tgg tgg ggt gat gtt tct gga gct ttg ctt 1008
Tyr Thr Gln Asn Pro Glu Trp Trp Gly Asp Val Ser Gly Ala Leu Leu
                325                 330                 335

cct cat ccg aga atg ctt atg atg ccc ggt gga aga ctt tct gat gga 1056
Pro His Pro Arg Met Leu Met Met Pro Gly Gly Arg Leu Ser Asp Gly
            340                 345                 350

tct agt gag aag aaa gac gtt tca agc aac acg gtc cag aca ttt acg 1104
Ser Ser Glu Lys Lys Asp Val Ser Ser Asn Thr Val Gln Thr Phe Thr
        355                 360                 365

gtt gta aca cct aag aat ggt gat tct ggt gac aaa gct tcg ttg aag 1152
Val Val Thr Pro Lys Asn Gly Asp Ser Gly Asp Lys Ala Ser Leu Lys
    370                 375                 380

ttt ttg atc tat ggt aag act ggt tgg ctt ggt ggt ctt cta ggg aaa 1200
Phe Leu Ile Tyr Gly Lys Thr Gly Trp Leu Gly Gly Leu Leu Gly Lys
385                 390                 395                 400

cta tgt gag aag caa ggg att aca tat gag tat ggg aaa gga cgt ctg 1248
Leu Cys Glu Lys Gln Gly Ile Thr Tyr Glu Tyr Gly Lys Gly Arg Leu
                405                 410                 415

gag gat aga gct tct ctt gtg gcg gat att cgt agc atc aaa cct act 1296
Glu Asp Arg Ala Ser Leu Val Ala Asp Ile Arg Ser Ile Lys Pro Thr
            420                 425                 430

cat gtg ttt aat gct gct ggt tta act ggc aga ccc aac gtt gac tgg 1344
His Val Phe Asn Ala Ala Gly Leu Thr Gly Arg Pro Asn Val Asp Trp
        435                 440                 445

tgt gaa tct cac aaa cca gag acc att cgt gta aat gtc gca ggt act 1392
Cys Glu Ser His Lys Pro Glu Thr Ile Arg Val Asn Val Ala Gly Thr
    450                 455                 460

ttg act cta gct gat gtt tgc aga gag aat gat ctc ttg atg atg aac 1440
Leu Thr Leu Ala Asp Val Cys Arg Glu Asn Asp Leu Leu Met Met Asn
465                 470                 475                 480

ttc gcc acc ggt tgc atc ttt gag tat gac gct aca cat cct gag ggt 1488
Phe Ala Thr Gly Cys Ile Phe Glu Tyr Asp Ala Thr His Pro Glu Gly
                485                 490                 495

tcg ggt ata ggt ttc aag gaa gaa gac aag cca aat ttc ttt ggt tct 1536
Ser Gly Ile Gly Phe Lys Glu Glu Asp Lys Pro Asn Phe Phe Gly Ser
            500                 505                 510

ttc tac tcg aaa acc aaa gcc atg gtt gag gag ctc ttg aga gaa ttt 1584
Phe Tyr Ser Lys Thr Lys Ala Met Val Glu Glu Leu Leu Arg Glu Phe
        515                 520                 525

gac aat gta tgt acc ttg aga gtc cgg atg cca atc tcc tca gac cta 1632
Asp Asn Val Cys Thr Leu Arg Val Arg Met Pro Ile Ser Ser Asp Leu

530                 535                 540

aac aac ccg aga aac ttc atc acg aag atc tcg cgc tac aac aaa gtg        1680
Asn Asn Pro Arg Asn Phe Ile Thr Lys Ile Ser Arg Tyr Asn Lys Val
545                 550                 555                 560

gtg gac atc ccg aac agc atg acc gta cta gac gag ctt ctc cca atc        1728
Val Asp Ile Pro Asn Ser Met Thr Val Leu Asp Glu Leu Leu Pro Ile
                565                 570                 575

tct atc gag atg gcg aag aga aac cta aga ggc ata tgg aat ttc acc        1776
Ser Ile Glu Met Ala Lys Arg Asn Leu Arg Gly Ile Trp Asn Phe Thr
            580                 585                 590

aac cca ggg gtg gtg agc cac aac gag ata ttg gag atg tac aag aat        1824
Asn Pro Gly Val Val Ser His Asn Glu Ile Leu Glu Met Tyr Lys Asn
        595                 600                 605

tac atc gag cca ggt ttt aaa tgg tcc aac ttc aca gtg gaa gaa caa        1872
Tyr Ile Glu Pro Gly Phe Lys Trp Ser Asn Phe Thr Val Glu Glu Gln
    610                 615                 620

gca aag gtc att gtt gct gct cga agc aac aac gaa atg gat gga tct        1920
Ala Lys Val Ile Val Ala Ala Arg Ser Asn Asn Glu Met Asp Gly Ser
625                 630                 635                 640

aaa cta agc aag gag ttc cca gag atg ctc tcc atc aaa gag tca ctg        1968
Lys Leu Ser Lys Glu Phe Pro Glu Met Leu Ser Ile Lys Glu Ser Leu
                645                 650                 655

ctc aaa tac gtc ttt gaa cca aac aag aga acc taa                        2004
Leu Lys Tyr Val Phe Glu Pro Asn Lys Arg Thr
            660                 665

<210>  10
<211>  667
<212>  PRT
<213>  Arabidopsis thaliana

<400>  10

Met Asp Asp Thr Thr Tyr Lys Pro Lys Asn Ile Leu Ile Thr Gly Ala
1               5                   10                  15

Ala Gly Phe Ile Ala Ser His Val Ala Asn Arg Leu Ile Arg Asn Tyr
            20                  25                  30

Pro Asp Tyr Lys Ile Val Val Leu Asp Lys Leu Asp Tyr Cys Ser Asp
        35                  40                  45

Leu Lys Asn Leu Asp Pro Ser Phe Ser Ser Pro Asn Phe Lys Phe Val
    50                  55                  60

Lys Gly Asp Ile Ala Ser Asp Asp Leu Val Asn Tyr Leu Leu Ile Thr
65                  70                  75                  80

Glu Asn Ile Asp Thr Ile Met His Phe Ala Ala Gln Thr His Val Asp

                85                  90                  95

Asn Ser Phe Gly Asn Ser Phe Glu Phe Thr Lys Asn Asn Ile Tyr Gly
            100                 105                 110

Thr His Val Leu Leu Glu Ala Cys Lys Val Thr Gly Gln Ile Arg Arg
        115                 120                 125

Phe Ile His Val Ser Thr Asp Glu Val Tyr Gly Glu Thr Asp Glu Asp
    130                 135                 140

Ala Ala Val Gly Asn His Glu Ala Ser Gln Leu Leu Pro Thr Asn Pro
145                 150                 155                 160

Tyr Ser Ala Thr Lys Ala Gly Ala Glu Met Leu Val Met Ala Tyr Gly
                165                 170                 175

Arg Ser Tyr Gly Leu Pro Val Ile Thr Thr Arg Gly Asn Asn Val Tyr
            180                 185                 190

Gly Pro Asn Gln Phe Pro Glu Lys Met Ile Pro Lys Phe Ile Leu Leu
        195                 200                 205

Ala Met Ser Gly Lys Pro Leu Pro Ile His Gly Asp Gly Ser Asn Val
    210                 215                 220

Arg Ser Tyr Leu Tyr Cys Glu Asp Val Ala Glu Ala Phe Glu Val Val
225                 230                 235                 240

Leu His Lys Gly Glu Ile Gly His Val Tyr Asn Val Gly Thr Lys Arg
                245                 250                 255

Glu Arg Arg Val Ile Asp Val Ala Arg Asp Ile Cys Lys Leu Phe Gly
            260                 265                 270

Lys Asp Pro Glu Ser Ser Ile Gln Phe Val Glu Asn Arg Pro Phe Asn
        275                 280                 285

Asp Gln Arg Tyr Phe Leu Asp Asp Gln Lys Leu Lys Lys Leu Gly Trp
    290                 295                 300

Gln Glu Arg Thr Asn Trp Glu Asp Gly Leu Lys Lys Thr Met Asp Trp
305                 310                 315                 320

Tyr Thr Gln Asn Pro Glu Trp Trp Gly Asp Val Ser Gly Ala Leu Leu
                325                 330                 335

Pro His Pro Arg Met Leu Met Met Pro Gly Gly Arg Leu Ser Asp Gly
        340                 345                 350

Ser Ser Glu Lys Lys Asp Val Ser Ser Asn Thr Val Gln Thr Phe Thr
    355                 360                 365

Val Val Thr Pro Lys Asn Gly Asp Ser Gly Asp Lys Ala Ser Leu Lys
    370                 375                 380

Phe Leu Ile Tyr Gly Lys Thr Gly Trp Leu Gly Gly Leu Leu Gly Lys
385                 390                 395                 400

Leu Cys Glu Lys Gln Gly Ile Thr Tyr Glu Tyr Gly Lys Gly Arg Leu
                405                 410                 415

Glu Asp Arg Ala Ser Leu Val Ala Asp Ile Arg Ser Ile Lys Pro Thr
        420                 425                 430

His Val Phe Asn Ala Ala Gly Leu Thr Gly Arg Pro Asn Val Asp Trp
    435                 440                 445

Cys Glu Ser His Lys Pro Glu Thr Ile Arg Val Asn Val Ala Gly Thr
    450                 455                 460

Leu Thr Leu Ala Asp Val Cys Arg Glu Asn Asp Leu Leu Met Met Asn
465                 470                 475                 480

Phe Ala Thr Gly Cys Ile Phe Glu Tyr Asp Ala Thr His Pro Glu Gly
                485                 490                 495

Ser Gly Ile Gly Phe Lys Glu Glu Asp Lys Pro Asn Phe Phe Gly Ser
        500                 505                 510

Phe Tyr Ser Lys Thr Lys Ala Met Val Glu Glu Leu Leu Arg Glu Phe
    515                 520                 525

Asp Asn Val Cys Thr Leu Arg Val Arg Met Pro Ile Ser Ser Asp Leu
    530                 535                 540

Asn Asn Pro Arg Asn Phe Ile Thr Lys Ile Ser Arg Tyr Asn Lys Val
545                 550                 555                 560

Val Asp Ile Pro Asn Ser Met Thr Val Leu Asp Glu Leu Leu Pro Ile
                565                 570                 575

Ser Ile Glu Met Ala Lys Arg Asn Leu Arg Gly Ile Trp Asn Phe Thr
        580                 585                 590

Asn Pro Gly Val Val Ser His Asn Glu Ile Leu Glu Met Tyr Lys Asn
        595                 600                 605

Tyr Ile Glu Pro Gly Phe Lys Trp Ser Asn Phe Thr Val Glu Glu Gln
    610                 615                 620

Ala Lys Val Ile Val Ala Ala Arg Ser Asn Asn Glu Met Asp Gly Ser
625                 630                 635                 640

Lys Leu Ser Lys Glu Phe Pro Glu Met Leu Ser Ile Lys Glu Ser Leu
                645                 650                 655

Leu Lys Tyr Val Phe Glu Pro Asn Lys Arg Thr
            660                 665

<210>  11
<211>  60
<212>  DNA
<213>  Stevia rebaudiana

<400>  11
gagtaaaatc tataacgaca ctaaggtgga aaaagaatat gtaagccaat tcgtagactt       60

<210>  12
<211>  33
<212>  DNA
<213>  Artificial

<220>
<223>  Artificial nucleic acid

<400>  12
cacccatatg gatgcaatgg ctacaactga gaa                                    33

<210>  13
<211>  32
<212>  DNA
<213>  Artificial

<220>
<223>  Artificial nucleic acid

<400>  13
agatctctag tttcttgcta gcacggtgat tt                                     32

<210>  14
<211>  35
<212>  DNA
<213>  Artificial

<220>
<223>  Artificial nucleic acid

<400>  14

cacccatatg gcggaacaac aaaagatcaa gaaat 35

<210> 15
<211> 35
<212> DNA
<213> Artificial

<220>
<223> Artificial nucleic acid

<400> 15
ggatccttaa gccttaatta gctcacttac aaatt 35

<210> 16
<211> 29
<212> DNA
<213> Artificial

<220>
<223> Artificial nucleic acid

<400> 16
cacccatatg gaaaataaaa cggagacca 29

<210> 17
<211> 32
<212> DNA
<213> Artificial

<220>
<223> Artificial nucleic acid

<400> 17
ggatccttac aacgatgaaa tgtaagaaac ta 32

<210> 18
<211> 33
<212> DNA
<213> Artificial

<220>
<223> Artificial nucleic acid

<400> 18
acagatctat ggatgcaatg gctacaactg aga 33

<210> 19
<211> 34
<212> DNA
<213> Artificial

<220>
<223> Artificial nucleic acid

<400> 19
tagtcgacta gtttcttgct agcacggtga tttc 34

<210>  20
<211>  42
<212>  DNA
<213>  Artificial

<220>
<223>  Artificial nucleic acid

<400>  20
aagcggccgc atgtacaacg ttacttatca tcaaaattca aa                          42


<210>  21
<211>  31
<212>  DNA
<213>  Artificial

<220>
<223>  Artificial nucleic acid

<400>  21
cgttaattaa ctctcatgat cgatggcaac c                                      31


<210>  22
<211>  34
<212>  DNA
<213>  Artificial

<220>
<223>  Artificial nucleic acid

<400>  22
aagcggccgc atggcggaac aacaaaagat caag                                   34


<210>  23
<211>  37
<212>  DNA
<213>  Artificial

<220>
<223>  Artificial nucleic acid

<400>  23
cgttaattaa gccttaatta gctcacttac aaattcg                                37


<210>  24
<211>  33
<212>  DNA
<213>  Artificial

<220>
<223>  Artificial nucleic acid

<400>  24
aaggatccat ggaaaataaa acggagacca ccg                                    33


<210>  25
<211>  44
<212>  DNA

<213>  Artificial

<220>
<223>  Artificial nucleic acid

<400>  25
gcgtcgactt acaacgatga aatgtaagaa actagagact ctaa                        44


<210>  26
<211>  33
<212>  DNA
<213>  Artificial

<220>
<223>  Artificial nucleic acid

<400>  26
ggatccatgg atgatactac gtataagcca aag                                    33


<210>  27
<211>  32
<212>  DNA
<213>  Artificial

<220>
<223>  Artificial nucleic acid

<400>  27
ctcgagttag gttctcttgt ttggttcaaa ga                                     32


<210>  28
<211>  22
<212>  DNA
<213>  Artificial

<220>
<223>  Artificial nucleic acid

<400>  28
caagtcccca accaaattcc gt                                                22


<210>  29
<211>  21
<212>  DNA
<213>  Artificial

<220>
<223>  Artificial nucleic acid

<400>  29
cacgaacccg tctggcaact c                                                 21


<210>  30
<211>  25
<212>  DNA
<213>  Artificial

<220>

<223>  Artificial nucleic acid

<400>  30
cccgtgtgat ttcttccact tgttc                                          25


<210>  31
<211>  21
<212>  DNA
<213>  Artificial

<220>
<223>  Artificial nucleic acid

<400>  31
caagaaccca tctggcaacg g                                              21


<210>  32
<211>  23
<212>  DNA
<213>  Artificial

<220>
<223>  Artificial nucleic acid

<400>  32
gctttgtcac cagaatcacc att                                            23


<210>  33
<211>  28
<212>  DNA
<213>  Artificial

<220>
<223>  Artificial nucleic acid

<400>  33
gattattaaa cttctttgcg tccatcca                                       28


<210>  34
<211>  32
<212>  DNA
<213>  Artificial

<220>
<223>  Artificial nucleic acid

<400>  34
cctctatact ttaacgtcaa ggagaaaaaa cc                                  32


<210>  35
<211>  39
<212>  DNA
<213>  Artificial

<220>
<223>  Artificial nucleic acid

<400>  35

tgccgcgcgg cagccatatg tacaacgtta cttatcatc 39

<210> 36
<211> 39
<212> DNA
<213> Artificial

<220>
<223> Artificial nucleic acid

<400> 36
gttagcagcc ggatccttaa ctctcatgat cgatggcaa 39

## Description

### TECHNICAL FIELD

The present invention relates to a novel steviol glycoside, a method for producing the same, and a sweetener composition containing the same. Furthermore, the present invention also relates to a food or beverage, a plant, an extract thereof and a flavor controlling agent containing the novel steviol glycoside.

### BACKGROUND ART

Leaves of *Stevia rebaudiana* contain a secondary metabolite called Steviol which is one type of diterpenoid, where steviol glycoside provides sweetness that is nearly 300 times the sweetness of sugar and is therefore utilized as a calorieless sweetener in the food industry. The demand for calorieless sweeteners is growing day by day as obesity has become a serious social problem worldwide and also for the sake of health promotion and reduction in the medical expenditure. Currently, aspartame and acesulfame potassium, which are artificially synthesized amino acid derivatives, are utilized as artificial sweeteners, but natural calorieless sweeteners like the steviol glycosides are expected to be safer and more likely to gain public acceptance.

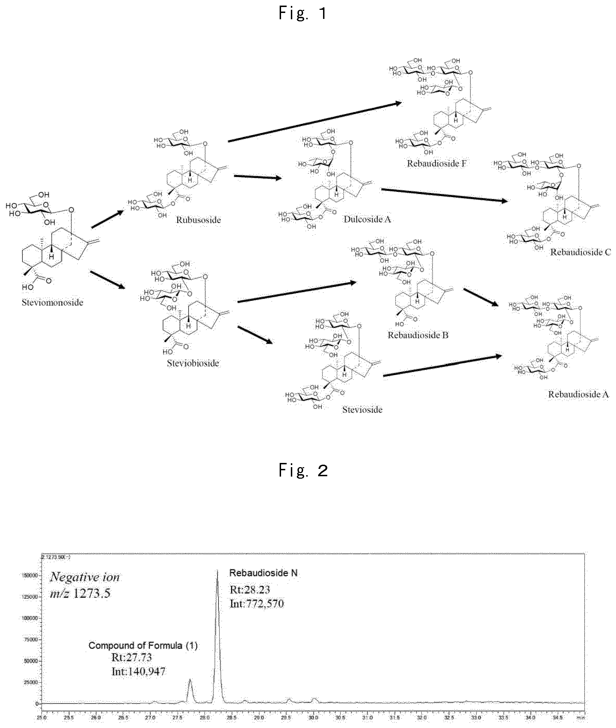

The major steviol glycosides from stevia are ultimately glycosylated to a glycoside named rebaudioside A (Reb.A) that has four sugar moieties (Figure 1). Stevioside, namely, a tri-glycosylated steviol glycoside and a precursor of Reb.A, is the most abundant glycoside. These two glycosides are the main substances responsible for the sweetness of stevia. Stevioside accounts for the largest content in stevia leaves and is known to provide sweetness that is about 250-300 times the sweetness of sugar. Reb.A is a tetra-glycosylated steviol glycoside that has strong sweetness (350-450 times sugar) with good taste quality. They have been drawing attention as calorieless sweeteners. Besides them, the existence of glycosides that are considered to be reaction intermediates and analogs having different types of sugar moieties is known. For example, while all of the four glycoside sugar moieties of Reb.A are glucose, rebaudioside C (Reb.C) is known to have rhamnose instead of glucose attached to C-2 of glucose at C-13, and rebaudioside F (Reb.F) is known to have xylose attached at the same position.

To date, attempts have been made to obtain a stevia plant having a higher Reb.A content than wild-type stevia plants by breeding or the like since the taste quality of Reb.A, in which all of the four glycoside sugar moieties are glucose, is good (for example, Patent Document 1).

### PRIOR ART DOCUMENT

### Patent document

Patent Document 1: Japanese Patent No. 3436317

### SUMMARY OF THE INVENTION

### Problem to be Solved by the Invention

Meanwhile, some stevia cultivars resulting from breeding may contain a minute amount of steviol glycoside whose structure is not yet identified, where the presence of such steviol glycoside present in minute quantity may potentially be contributing to the flavor characteristic of the stevia extract. Moreover, although researches have been made thus far on steviol glycosides obtained by further attaching glucose to Reb.A and on cultivars containing the same, not much research has been made at this point on cultivars containing an abundant amount of a steviol glycoside having rhamnose like Reb.C and on such a steviol glycoside.

Accordingly, the objective of the present invention is to determine the structure of a novel steviol glycoside present in minute quantity that affects the taste quality, and to identify the characteristics of its taste quality. In addition, further objectives of the present invention are to provide a novel steviol glycoside, a method for producing the same, and a sweetener composition containing the same.

### Means for Solving the Problem

The present inventors have gone through extensive investigation to solve the above-described problem, and as a result of which succeeded in determining the structure of the novel steviol glycoside present in minute quantity that affects the taste quality. The present invention was made based on the above-described finding.

### EFFECT OF THE INVENTION

The present invention can provide a novel steviol glycoside present in minute quantity that affects the taste quality. Furthermore, the present invention can also provide a method for producing the novel steviol glycoside, and a sweetener composition, a food or beverage, a plant, an extract thereof and a flavor controlling agent containing the novel steviol glycoside.

### BRIEF DESCRIPTION OF DRAWINGS

Figure 1 illustrates a diagram showing structures and names of steviol glycosides.
Figure 2 illustrates a diagram showing a selected ion chromatogram of Sample 1 at m/z of 1273.5.
Figure 3 illustrates diagrams showing MS/MS and MS³ fragmented mass spectra of rebaudioside N and the compound represented by Formula (1).

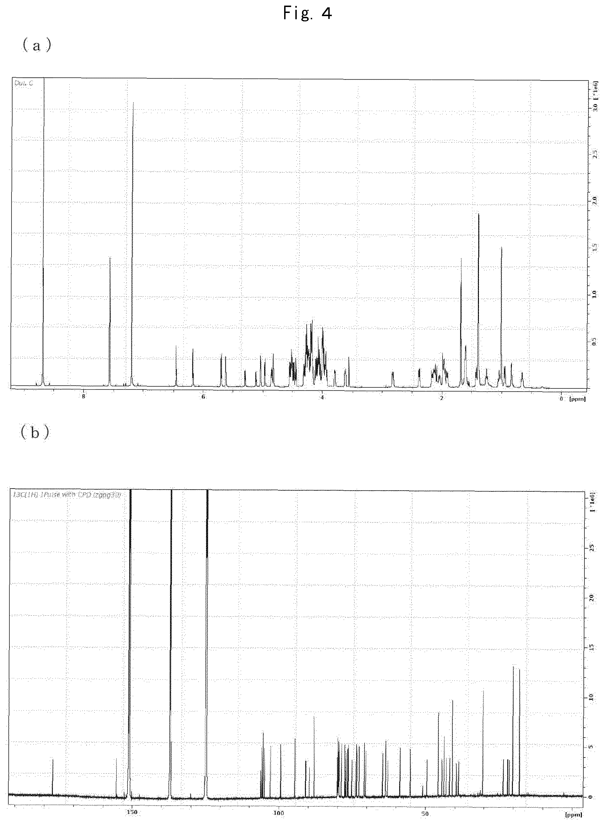

Figure 4 illustrates (a) a diagram showing a ¹H-NMR spectrum of Compound 11 (800MHz, Pyr-d5); and (b) a diagram showing a ¹³C-NMR spectrum of Compound 11 (200MHz, Pyr-d5).

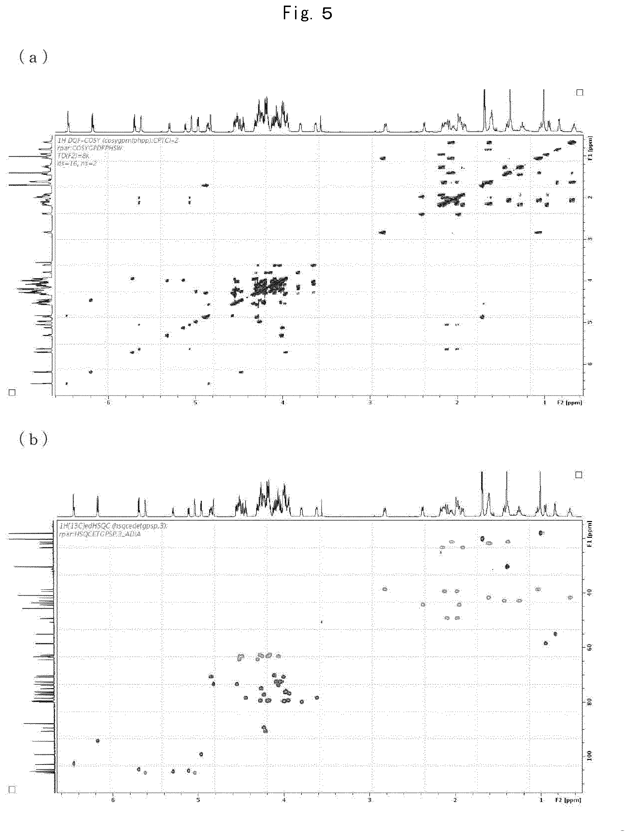

Figure 5 illustrates (a) a diagram showing a ¹H-¹H cosy spectrum of Compound 11 (800MHz, Pyr-d5); and (b) a diagram showing a HSQC spectrum of Compound 11 (800MHz, Pyr-d5).
Figure 6 illustrates diagrams showing a procedure for identifying a novel steviol glycoside contained in an extract of a plant.
Figure 7 illustrates a diagram showing a selected ion chromatogram of a sample obtained by biosynthesis at m/z of 1273.5.
Figure 8 illustrates diagrams showing results of sensory evaluations for comparison between the novel steviol glycoside and rebaudioside A.
Figure 9 illustrates graphs showing results from evaluating an effect of the flavor controlling agent of the present invention to improve the lingering aftertaste of Reb.A.
Figure 10 illustrates graphs showing results from evaluating an effect of a flavor controlling agent of the present invention to improve the lingering aftertaste of Reb.D.
Figure 11 illustrates graphs showing results from evaluating an effect of the flavor controlling agent of the present invention to enhance sweetness of sugar (sucrose).

### MODES FOR CARRYING OUT THE INVENTION

Hereinafter, the present invention will be described in detail. The following embodiments are provided for illustrating the present invention and not with the intention of limiting the present invention solely to these embodiments. The present invention may be carried out in various modes without departing from the scope thereof. All of the documents, publications, patent publications and other patent documents cited herein are incorporated herein by reference.

The terms "rebaudioside" and "Reb." as used herein have the same meaning and both refer to "rebaudioside". Similarly, the terms "dulcoside" and "dulcoside" as used herein have the same meaning and both refer to "dulcoside".

### 1. Novel steviol glycoside

For the first time, the present inventors identified the structure of a minute amount of a novel steviol glycoside that affects taste quality. The novel steviol glycoside of the present invention (hereinafter, also referred to as the "glycoside of the present invention") is a compound represented by Formula (1): 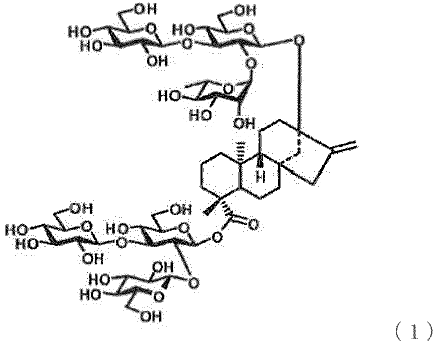 or a derivative, a salt or a hydrate thereof.

As represented above, the glycoside of the present invention has a sugar chain containing three glucose moieties at C-19 of steviol and a sugar chain containing two glucose moieties and one rhamnose moiety at C-13.

The glycoside of the present invention may be not only the compound represented by Formula (1) but may also be a derivative, a salt or a hydrate thereof. The term "derivative" as used herein refers to a compound resulting from a structural change of a minor moiety of the compound, for example, a compound in which some of the hydroxyl groups are substituted with other substituents. Therefore, derivatives of the compound represented by Formula (1) include compounds in which some of the hydroxyl groups contained in the compound have been substituted with a substituent selected from hydrogen, a halogen, an alkyl group, an alkenyl group, an alkynyl group, an aryl group, an amino group, a cyano group or the like. As used herein, a "salt of the compound represented by Formula (1)" refers to a physiologically acceptable salt, for example, a sodium salt, of the compound represented by Formula (1). Furthermore, a "hydrate of the compound represented by Formula (1)" as used herein refers to a compound resulting from addition of a water molecule to the compound represented by Formula (1).

While the glycoside of the present invention is not particularly limited, it may be a plant-derived product, a chemically synthesized product or a biosynthetic product. For example, it may be isolated and purified from a plant with abundant Reb.C, or it may be obtained by chemical synthesis or biosynthesis. Details of a method for producing a glycoside of the present invention will be described later herein.

The glycoside of the present invention is sweeter than sugar (sucrose), has taste quality of good lingering sweet aftertaste, and can affect the taste quality of foods/beverages in a small amount. Thus, the glycoside of the present invention can be used as a novel sweetener.

Moreover, in another aspect of the present invention, the novel steviol glycoside of the present invention is a compound represented by Formula (A): 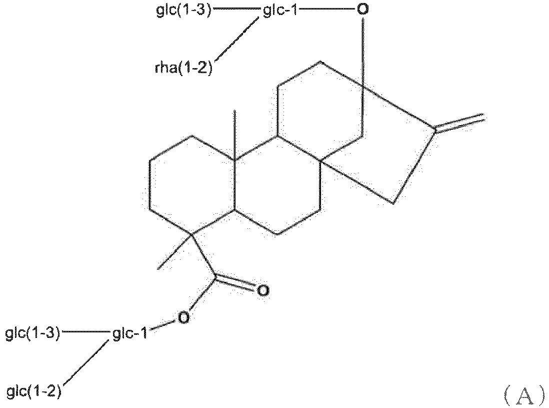 or a derivative, a salt or a hydrate thereof.

### 2. Sweetener composition containing novel steviol glycoside

In one aspect of the present invention, a sweetener composition containing the compound represented by Formula (1), or a derivative, a salt or a hydrate thereof (hereinafter, also referred to as the "sweetener composition of the present invention") is provided. The sweetener composition of the present invention is not particularly limited as long as it contains the compound represented by Formula (1), or a derivative, a salt or a hydrate thereof, and it may be a composition containing an extract containing the compound represented by Formula (1), or a derivative, a salt or a hydrate thereof.

The amount of the glycoside of the present invention contained in the sweetener composition of the present invention is not particularly limited.

Alternatively, the sweetener composition of the present invention is preferably a composition containing the glycoside of the present invention in a larger amount than the amount in a wild-type stevia or stevia extract by at least 0.01%. As mentioned above, the glycoside of the present invention was detected for the first time in a cultivar containing abundant Reb.C, and it is not contained in a wild-type stevia or an extract thereof at all or, if any, contained in an amount at the detection limit or below.

The sweetener composition of the present invention may further contain other steviol glycosides. For example, the sweetener composition of the present invention may contain, in addition to the glycoside of the present invention, one or more types of steviol glycosides selected from the group consisting of rebaudioside A, rebaudioside B, rebaudioside C, rebaudioside D, rebaudioside E, rebaudioside F, rebaudioside I, rebaudioside J, rebaudioside K, rebaudioside N, rebaudioside M, rebaudioside O, rebaudioside Q, rebaudioside R, dulcoside A, rubusoside, steviol, steviol monoside, steviol bioside and stevioside.

In a case where other steviol glycoside is contained, the composition ratio of the glycoside of the present invention and other steviol glycoside is preferably 0.01:9.99-6:4 in a mass ratio.

The sweetener composition of the present invention may further contain a general sweetener. Examples of such a general sweetener include natural sweeteners such as fructose, sugar, fructose-glucose syrup, glucose, maltose, sucrose, high-fructose syrup, sugar alcohol, oligosaccharide, honey, pressed sugarcane juice (brown sugar syrup), starch syrup, Lo Han Kuo (*Siraitia grosvenorii*) powder, Lo Han Kuo extract, licorice powder, licorice extract, *Thaumatococcus daniellii* seed powder and *Thaumatococcus daniellii* seed extract, and artificial sweeteners such as acesulfame potassium, sucralose, neotame, aspartame and saccharin. Among them, natural sweeteners are preferably used from the aspect of imparting clean taste, easy drinkability, natural flavor and moderately rich taste, where fructose, glucose, maltose, sucrose and sugar are particularly preferably used. Either a single type or a plurality of types of these sweetness ingredients may be used.

### 3. Food or beverage containing novel steviol glycoside

In one aspect of the present invention, a food or beverage containing the compound represented by Formula (1), or a derivative, a salt or a hydrate thereof (hereinafter, also referred to as the "food or beverage of the present invention") is provided. The food or beverage of the present invention is not particularly limited as long as it contains the compound represented by Formula (1), or a derivative, a salt or a hydrate thereof, and it may be a food or beverage containing an extract containing the compound represented by Formula (1), or a derivative, a salt or a hydrate thereof. As used herein, a food or beverage refers to foods and beverages. Therefore, in some embodiments, the present invention provides a novel food or beverage, and a method for producing said food or beverage.

While the amount of the glycoside of the present invention contained in the food or beverage of the present invention differs depending on the specific food or beverage, it is preferably around 0.0004%-0.8% and particularly preferably 0.04%-0.4%. As long as the content lies within this range, the lingering aftertaste can advantageously be suppressed.

The food or beverage of the present invention may further contain other steviol glycosides. For example, the sweetener composition of the present invention may contain, in addition to the glycoside of the present invention, one or more types of steviol glycosides selected from the group consisting of rebaudioside A, rebaudioside B, rebaudioside C, rebaudioside D, rebaudioside E, rebaudioside F, rebaudioside I, rebaudioside J, rebaudioside K, rebaudioside N, rebaudioside M, rebaudioside O, rebaudioside Q, rebaudioside R, dulcoside A, rubusoside, steviol, steviol monoside, steviol bioside and stevioside.

In a case where other steviol glycoside is contained, the composition ratio of the glycoside of the present invention and other steviol glycoside is preferably 0.01:9.99-6:4 in a mass ratio.

The food or beverage of the present invention may further contain a general sweetener. Examples of such a general sweetener include natural sweeteners such as fructose, sugar, fructose-glucose syrup, glucose, maltose, sucrose, high-fructose syrup, sugar alcohol, oligosaccharide, honey, pressed sugarcane juice (brown sugar syrup), starch syrup, Lo Han Kuo (*Siraitia grosvenorii)* powder, Lo Han Kuo extract, licorice powder, licorice extract, *Thaumatococcus daniellii* seed powder and *Thaumatococcus daniellii* seed extract, and artificial sweeteners such as acesulfame potassium, sucralose, neotame, aspartame and saccharin. Among them, natural sweeteners are preferably used from the aspect of imparting clean taste, easy drinkability, natural flavor and moderately rich taste, where fructose, glucose, maltose, sucrose and sugar are particularly preferably used. Either a single type or a plurality of types of these sweetness ingredients may be used.

Examples of the food of the present invention include, but not particularly limited to, a confection, a bread, cereal flour, noodles, rice, a processed agricultural/forestry food, a processed livestock product, a processed fishery product, a milk/dairy product, an oil-and-fat/processed oil-and-fat product, seasoning or other food material.

Examples of the beverage of the present invention include, but not particularly limited to, a carbonated beverage, a non-carbonated beverage, an alcoholic beverage, a non-alcoholic beverage, a coffee beverage, a tea beverage, a cocoa beverage, a nutritious beverage and a functional beverage.

The beverage of the present invention may be heat sterilized and packaged to be prepared as a packaged beverage. Examples of such package include, but not particularly limited to, a PET bottle, an aluminum can, a steel can, a paper package, a chilled cup, and a bottle. In a case where heat sterilization is to be performed, the type of heat sterilization is not particularly limited. For example, heat sterilization may be performed by employing a common technique such as UHT sterilization, retort sterilization or the like. While the temperature during the heat sterilization process is not particularly limited, it is, for example, 65-130°C, and preferably 85-120°C, for 10-40 minutes. Sterilization, however, can be carried out at an appropriate temperature for a several seconds, for example, 5-30 seconds, without problem as long as the same sterilizing value as that under the above-described conditions can be earned.

### 4. Plant containing novel steviol glycoside and extract thereof

In one aspect of the present invention, a plant containing the novel steviol glycoside and an extract thereof are provided. Furthermore, in another aspect of the present invention, a food or beverage, preferably a beverage, containing the plant of the present invention or an extract of the plant is provided. While the amount of the glycoside of the present invention contained in the plant of the present invention is not particularly limited, it is preferably 0.001%-1.000% and more preferably 0.01%-0.80%.

Preferably, the plant of the present invention is a plant that contains the glycoside of the present invention in a larger amount than a wild-type stevia species by 0.01% or more. As described above, the steviol glycoside of the present invention is not contained in a wild-type stevia at all or, if any, contained in an amount of the detection limit or less.

The phrase "contains the glycoside of the present invention in a larger amount than a wild-type stevia species by 0.01% or more" means that, with respect to an amount (concentration) of the glycoside of the present invention contained per unit quantity (e.g. 10 ml) of a liquid extract from fresh leaves (undried leaves) of a wild-type stevia plant, an amount (concentration) of the glycoside of the present invention contained in an equal unit quantity of a liquid extract from fresh leaves (undried leaves) of the plant of the present invention (the same amount as that of the liquid extract from the leaves of the wild-type stevia plant) is higher by 0.01% or more. Here, the plant of the present invention may contain the glycoside of the present invention in a larger amount than a wild-type stevia species by 0.02% or more, 0.03% or more, 0.04% or more, 0.05% or more, 0.07% or more, 0.09% or more, 0.10% or more, 0.15% or more, 0.20% or more, 0.40% or more, 0.60% or more, 0.80% or more, 1.0% or more, 1.50% or more, 2.00% or more, 4.00% or more, 6.00% or more, 8.00% or more, or 10.00% or more.

Moreover, the phrase "the proportion of the glycoside of the present invention among the total steviol glycosides is 0.01% or more" means that the glycoside of the present invention exists at a percentage of 0.01% or more with respect to the total content of the steviol glycosides existing in the liquid extract from the fresh leaves (undried leaves) of the stevia plant of the present invention. Here, the total steviol glycosides neither contain unknown steviol glycosides nor any steviol glycoside existing in an amount less than the detection limit. Preferably, the total steviol glycosides consist of rebaudioside A, rebaudioside B, rebaudioside C, rebaudioside D, rebaudioside E, rebaudioside F, rebaudioside I, rebaudioside J, rebaudioside K, rebaudioside N, rebaudioside M, rebaudioside O, rebaudioside Q, rebaudioside R, dulcoside A, rubusoside, steviol, steviol monoside, steviol bioside and stevioside.

While the content of the glycoside of the present invention in the plant of the present invention is as described above, in a case where dried leaves are obtained from the plant of the present invention, the glycoside of the present invention may exist in an amount of 0.01 wt% or more, 0.02 wt% or more, 0.03 wt% or more, 0.04 wt% or more, 0.05 wt% or more, 0.07 wt% or more, 0.10 wt% or more, 0.15 wt% or more, 0.20 wt% or more, 0.30 wt% or more, 0.50 wt% or more, 0.60 wt% or more, 0.80 wt% or more, 1.00 wt% or more, 2.00 wt% or more, 4.00 wt% or more, 6.00 wt% or more, 8.00 wt% or more, or 10.00 wt% or more with respect to the weight of said dried leaves.

Here, dried leaves of the plant of the present invention refer to those obtained by drying fresh leaves of the plant of the present invention to reduce their water content to 10 wt% or less, 7 wt% or less, 5 wt% or less, 4 wt% or less, 3 wt% or less, 2 wt% or less, or 1 wt% or less. Preferably, the water content of the dried leaves of the plant of the present invention is 3-4 wt%.

Examples of the plant of the present invention include plants with abundant Reb.C. As described above, the steviol glycoside of the present invention is not contained in a wild-type stevia or an extract thereof at all or, if any, contained in an amount of the detection limit or less. Meanwhile, the present inventors found out that the steviol glycoside of the present invention is contained in a larger amount in a plant having abundant Reb.C. Therefore, the novel steviol glycoside and the extract thereof also comprise such a plant with abundant Reb.C and an extract thereof.

Examples of such a plant with abundant Reb.C include, but are not particularly limited to, high-rebaudioside C-containing non-recombinant stevia plants which contain rebaudioside C in a larger amount than a wild-type stevia species by 20% or more, and whose proportion of rebaudioside C among the total steviol glycosides is 40% or more (hereinafter, also referred to as a "high-Reb.C plant").

Examples of such a high-Reb.C plant include high-rebaudioside C-containing non-recombinant stevia plants which contain rebaudioside C in a larger amount than a wild-type stevia species by 20% or more, and whose proportion of rebaudioside C among the total steviol glycosides is 40% or more.

A high-Reb.C plant is a cultivar derived from a plant of a wild-type stevia, in which genetic mutation has occurred to increase rebaudioside C. Examples of such genetic mutation include, but not particularly limited to, genetic mutation induced under naturally occurring conditions, genetic mutation induced by a non-recombinational technique and genetic mutation induced by genetic recombination.

A high-Reb.C plant can be screened, for example, by detecting gene polymorphism in the tissue of the plant. Herein, "screening" means to identify a high-Reb.C plant among other plants and to select the high-Reb.C plant.

The high-Reb.C plant may also be screened according to a screening method that includes a step of identifying a polymorphism of A in the wild type being altered to T at the 60th nucleotide of the nucleotide sequence represented by SEQ ID NO:11 in the genome of a test plant.

A plant of the present invention not only comprises whole plants but may also include plant organs (for example, leaf, petal, stem, root, seed, etc.), plant tissues (for example, epidermis, phloem, parenchyma, xylem, vascular bundles, palisade tissue, spongy tissue, etc.), various forms of plant cells (for example, suspension cultured cells), a protoplast, a leaf piece, callus and the like.

In addition, a plant of the present invention may also comprise a tissue culture or a plant cell culture. This is because such a tissue culture or plant cell culture may be cultured to regenerate a plant. Examples of the tissue culture or the plant cell culture of the plant of the present invention include, but not limited to, an embryo, meristematic cells, pollen, a leaf, a root, a root apex, a petal, a protoplast, a leaf piece and callus.

An extract of a plant of the present invention can be obtained by reacting a fresh leaf or a dried leaf of the plant of the present invention with an appropriate solvent (an aqueous solvent such as water or an organic solvent such as alcohol, ether or acetone). For conditions for extraction, see a method described in WO2016/090460 or a method described in the example below.

Preferably, the extract of the plant of the present invention contains the glycoside of the present invention in a larger amount than a wild-type stevia by 0.01% or more, where the proportion of the glycoside of the present invention among the total steviol glycosides is 0.01% or more. Here, the phrase "contains the glycoside of the present invention in a larger amount than a wild-type stevia by 0.01% or more" means the same as described above. Similarly, the phrase the "proportion of the glycoside of the present invention among the total steviol glycosides is 0.01% or more" also means the same as described above.

### 5. Flavor controlling agent containing novel steviol glycoside

Although the novel steviol glycoside of the present invention is contained in a stevia extract in a minute quantity, it is considered to have an influence on the flavor of the stevia extract. While not wishing to be bound by any theory, addition of a small amount of the steviol glycoside of the present invention is presumed to be capable of controlling the flavor of a food or beverage. Therefore, in one aspect of the present invention, a flavor controlling agent containing the above-described compound represented by Formula (1) or a derivative, a salt or a hydrate thereof is provided.

As used herein, a "flavor controlling agent" refers to a substance that can be added to a food or beverage to control the flavor of the food or beverage. Preferably, the flavor controlling agent of the present invention can be added to a food or beverage so as to control the flavor of the food or beverage itself without the consumers recognizing the taste of the flavor controlling agent itself. For example, since the steviol glycoside of the present invention has good lingering sweet aftertaste as compared to conventional steviol glycosides, it can be used as a flavor controlling agent for controlling the lingering sweet aftertaste of the food or beverage.

The flavor controlling (enhancing) agent of the present invention preferably contains, in addition to the above-described compound represented by Formula (1) or a derivative, a salt or a hydrate thereof, one or more types of other sweeteners. Examples of such sweetener include: one or more types of steviol glycosides selected from the group consisting of rebaudioside A, rebaudioside D, rebaudioside B, rebaudioside M, rebaudioside N, rebaudioside O, rebaudioside E, rebaudioside K and rebaudioside J; natural sweeteners such as fructose, sugar, fructose-glucose syrup, glucose, maltose, sucrose, high-fructose syrup, sugar alcohol, oligosaccharide, honey, pressed sugarcane juice (brown sugar syrup), starch syrup, Lo Han Kuo (*Siraitia grosvenorii*) powder, Lo Han Kuo extract, licorice powder, licorice extract, *Thaumatococcus daniellii* seed powder and *Thaumatococcus daniellii* seed extract; and artificial sweeteners such as acesulfame potassium, sucralose, neotame, aspartame and saccharin.

In one aspect of the present invention, the flavor controlling agent of the present invention is a flavor controlling agent that improves lingering aftertaste and that contains the compound represented by Formula (1) or a derivative, a salt or a hydrate thereof. While Brix of commercially available foods or beverages (for example, refreshing beverages) is usually up to around 15, reduction in the amount of sugar in foods or beverages has been considered due to the recent growth of health consciousness and the introduction of sugar tax. Where the amount of sugar is reduced, use of a non-sugar sweetener (for example, a non-calorie sweetener) has been attempted to compensate for the loss of Brix due to the sugar reduction. For example, if the amount of sugar in a food or beverage originally having Brix of 10 is reduced by half, Brix will be 5. Therefore, there is a need to add a non-sugar sweetener to make up for the sweetness level corresponding to Brix of 5. Many of the non-sugar sweeteners, however, have unique flavors that differ from sugar, where one of such characteristic flavors is bad lingering sweet aftertaste. Since the steviol glycoside of the present invention has good lingering sweet aftertaste, a flavor controlling agent containing the steviol glycoside of the present invention can be used as a flavor controlling agent for improving the lingering aftertaste. Herein, "Brix" is a scale of a sweetness level of a food or beverage, that is, a value of the concentration of the soluble solid content expressed in a weight percent concentration in a sucrose solution at 20°C. Accordingly, it is represented by an amount of sucrose (g) in 100 g of an aqueous sucrose solution. For example, Brix of 5 represents a sweetness level corresponding to the sweetness level of 5 g of sucrose in 100 g of an aqueous sucrose solution.

The flavor controlling agent of the present invention is preferably added to the non-sugar sweetener contained in a food or beverage in an amount of 1 mass%-15 mass% based on the mass of the sweetener. The flavor controlling agent of the present invention is added more preferably in an amount of 1.5 mass%-12 mass%, and still more preferably in an amount of 3.5 mass%-11 mass% based on the mass of the sweetener. The content of the non-sugar sweetener in the food or beverage added with the flavor controlling agent of the present invention is preferably 5-13, more preferably 5-12 and still more preferably 5-7 in terms of Brix. Here, the phrase "the content of the non-sugar sweetener is 5 in terms of Brix" refers to a content that gives the sweetness of the aqueous solution containing the non-sugar sweetener to be equivalent to Brix of 5. For example, if the sweetness level of a non-sugar sweetener is sweeter than sugar by 200 times, the amount that gives "the content of the non-sugar sweetener to be 5 in terms of Brix" is 0.025 g in 100 g of an aqueous solution containing the non-sugar sweetener. In a preferable aspect of the present invention, a flavor controlling agent of the present invention is added to the non-sugar sweetener contained in the food or beverage in an amount of 1 mass%-15 mass% based on the mass of the sweetener, wherein the content of the non-sugar sweetener is 5.5-12 in terms of Brix. In other preferable aspect of the present invention, the flavor controlling agent of the present invention is added to the non-sugar sweetener contained in the food or beverage in an amount of 1.5 mass%-12 mass% based on the mass of the sweetener, wherein the content of the non-sugar sweetener is 5-13 in terms of Brix.

Examples of other sweetener contained in the food or beverage include, but not limited to: one or more types of steviol glycoside selected from the group consisting of rebaudioside A, rebaudioside D, rebaudioside B, rebaudioside M, rebaudioside N, rebaudioside O, rebaudioside E, rebaudioside K and rebaudioside J; natural sweeteners such as fructose, fructose-glucose syrup, glucose, maltose, high-fructose syrup, sugar alcohol, oligosaccharide, honey, pressed sugarcane juice (brown sugar syrup), starch syrup, Lo Han Kuo (*Siraitia grosvenorii)* powder, Lo Han Kuo extract, licorice powder, licorice extract, *Thaumatococcus daniellii* seed powder and *Thaumatococcus daniellii* seed extract; and artificial sweeteners such as acesulfame potassium, sucralose, neotame, aspartame and saccharin.

In another aspect of the present invention, a flavor controlling agent of the present invention is a flavor controlling agent containing the above-described compound represented by Formula (1) or a derivative, a salt or a hydrate thereof for enhancing sweetness. A flavor controlling agent for enhancing sweetness refers to a flavor controlling agent that can be added to a food or beverage containing a sweetener so that it can impart stronger sweetness to the food or beverage than a simple sum of the sweetness of the flavor controlling agent thereto. For example, when a flavor controlling agent of an amount equivalent to Brix of 0.1 is added to a food or beverage with sweetness equivalent to Brix of 9, the flavor controlling agent should be capable of imparting a sweetness level exceeding Brix of 9.1 (for example, Brix of 9.2) to the food or beverage. Use of such a flavor controlling agent for enhancing sweetness can reduce the total amount of the sweeteners used, and thus advantageous in realizing calorie reduction and cost reduction.

If the sweetness level of the sweetener targeted for sweetness enhancement is 1-10, the flavor controlling agent for enhancing sweetness of the present invention is preferably added in an amount of 0.05 mass%-2.0 mass%, more preferably added in an amount of 0.1 mass%-1.5 mass%, and still more preferably added in an amount of 0.2 mass%-1.2 mass% based on the mass of the sweetener targeted for sweetness enhancement.

Examples of the sweetener targeted for sweetness enhancement include, but not particularly limited to: one or more types of steviol glycoside selected from the group consisting of rebaudioside A, rebaudioside D, rebaudioside B, rebaudioside M, rebaudioside N, rebaudioside O, rebaudioside E, rebaudioside K and rebaudioside J; natural sweeteners such as fructose, sugar, fructose-glucose syrup, glucose, maltose, sucrose, high-fructose syrup, sugar alcohol, oligosaccharide, honey, pressed sugarcane juice (brown sugar syrup), starch syrup, Lo Han Kuo (*Siraitia grosvenorii*) powder, Lo Han Kuo extract, licorice powder, licorice extract, *Thaumatococcus daniellii* seed powder and *Thaumatococcus daniellii* seed extract; and artificial sweeteners such as acesulfame potassium, sucralose, neotame, aspartame and saccharin.

### 6. Method for producing novel steviol glycoside

As described above, the steviol glycoside of the present invention can be produced by (A) isolation/purification from a plant, (B) a chemical synthesis, or (C) a biosynthesis. Hereinafter, each of them will be described.

### (A) Isolation/purification from plant

Since the plant of the present invention contains the novel steviol glycoside of the present invention, the novel steviol glycoside can be isolated/purified from said plant. A fresh or dried leaf of the plant of the present invention is allowed to react with an appropriate solvent (an aqueous solvent such as water or an organic solvent such as alcohol, ether or acetone) to extract the novel steviol glycoside in a liquid extract state. For extraction conditions and else, see the method described in WO2016/090460 or the method described in the example below.

Furthermore, the resulting liquid extract may be subjected to a known method such as a gradient of ethyl acetate or other organic solvent: water, high performance liquid chromatography (HPLC), or ultra (high) performance liquid chromatography (UPLC) to isolate/purify the novel steviol glycoside.

The content of the novel steviol glycoside in the plant can be determined by the method described in WO2016/090460 or the method described in the example below. Specifically, the content can be measured by sampling fresh leaves from the plant of the present invention and subjecting the leaves to LC-MS/MS.

### (2) Chemical synthesis

A method for synthesizing the steviol glycoside of the present invention will be described in detail hereinbelow.

Steviol glycosides have structures in which different sugar moieties (glucose, rhamnose, xylose, etc.) are attached to the aglycone, i.e. steviol, via various linkage forms (linkage positions and conformations). Therefore, a steviol glycoside of interest can be obtained via various pathways depending on the selected starting material. Those skilled in the art to which the present invention pertains, however, wound understand that the time and the yield for obtaining the compound of interest greatly vary depending on the synthetic pathways.

This time, the present inventors found out a novel method for producing a steviol glycoside of the present invention with higher selectivity and higher yield via a specific synthetic pathway. According to the method for synthesizing the steviol glycoside of the present invention, a chemical synthesis of the steviol glycoside proceeds by separating the steviol glycoside into a "steviol glycoside" and a "sugar hemiacetal form" as shown in Scheme 1.

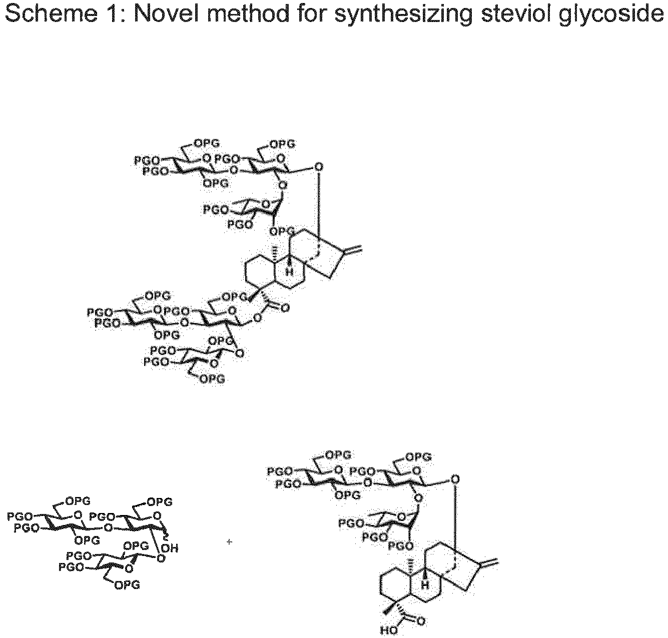

The steviol glycoside can be prepared by deriving from an existing natural substance (rebaudioside, dulcoside, stevioside, steviol bioside, rubusoside, etc.). Meanwhile, the sugar hemiacetal form can be prepared either from an existing natural substance or by a chemical synthesis. The present inventors found that the steviol glycoside of interest can be obtained with good yield and extremely high β-selectivity by condensing the steviol glycoside and the sugar hemiacetal form through the Mitsunobu reaction.

In one aspect of the present invention, a method for producing the compound represented by Formula (1) is provided, where the method comprises the steps of:
(A) synthesizing a compound represented by Formula (3) below: 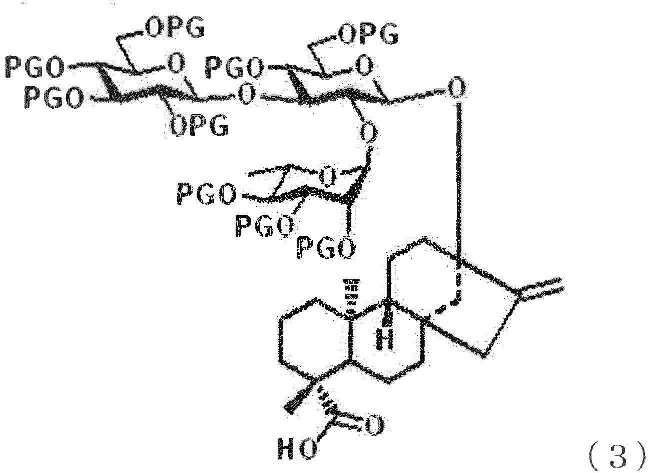 (wherein, PGs each independently represents a protecting group) from rebaudioside C represented by Formula (2) below: 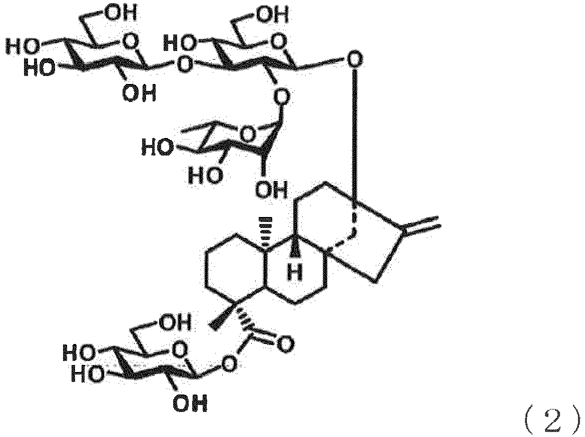 ; and
(B) synthesizing a compound represented by Formula (4) below 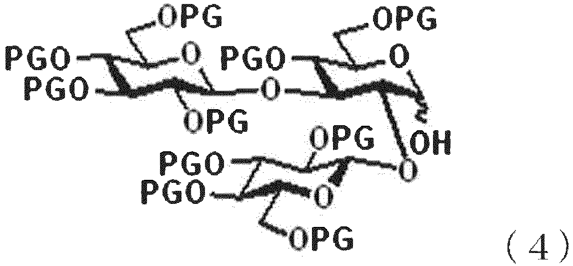 (wherein, PGs each independently represents a protecting group) from a glucopyranoside derivative.

In another aspect of the present invention, the method for producing the compound represented by Formula (1) is provided, where the method further comprises a step of allowing reaction between the compound represented by Formula (3) above and the compound represented by Formula (4) above in the presence of a phosphine reagent and an azo compound to obtain a compound represented by Formula (5) below 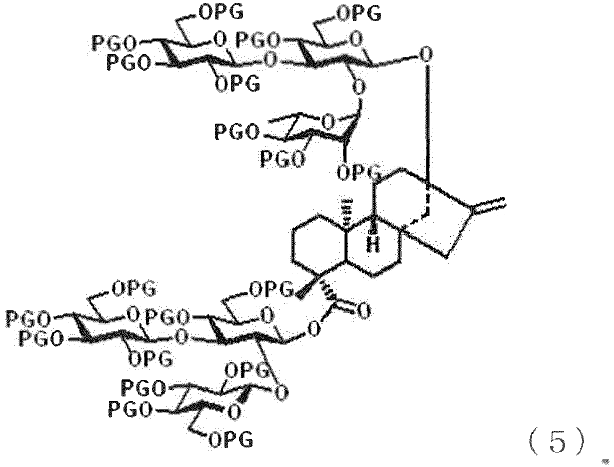 (wherein, PGs each independently represents a protecting group).

Herein, examples of the protecting group include an acyl protecting group, a trisubstituted silyl group, an acetal protecting group and an ether protecting group. Preferable examples include a trisubstituted silyl group (a trimethylsilyl group, a triethylsilyl group, a t-butyldimethylsilyl group, etc.) and an acyl protecting group (an acetyl group, a benzoyl group, etc.).

### (A) First step (synthesis of steviol glycoside)

A steviol glycoside can be obtained, for example, by following Scheme 2 below using naturally occurring rebaudioside C (dulcoside B) as a raw material.

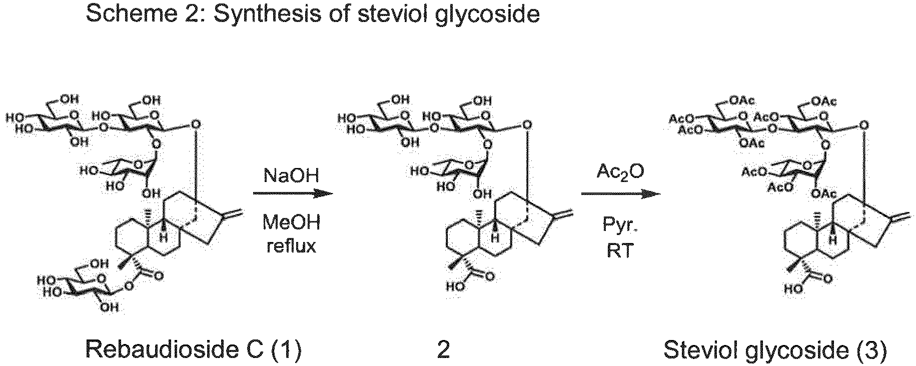

First, rebaudioside C is dissolved in a solvent such as methanol and water, added with a strong base such as sodium hydroxide, and refluxed at 60°C-120°C for 2 hours or longer so that the glucose molecule is removed from C-19 of rebaudioside C to give Compound 2 above. In doing so, the solvent may be evaporated after neutralizing the reaction solution with a cation exchange resin or the like.

Compound 2 is further dissolved in a solvent such as pyridine, and added with acetic anhydride or the like to protect the hydroxyl groups contained in Compound 2, thereby obtaining Compound 3.

### (B) Second step (synthesis of trisaccharide hemiacetal)

The trisaccharide hemiacetal can be obtained, for example, by following Scheme 3 below using a commercially available glucopyranoside derivative as a raw material.

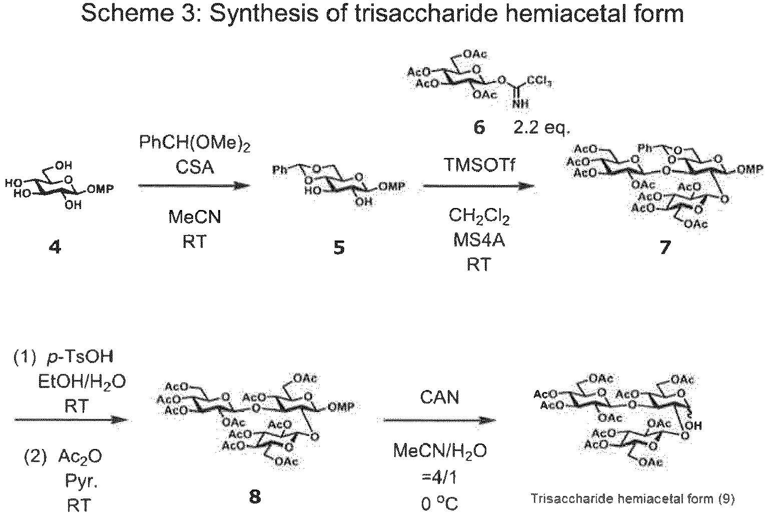

First, 4-methoxyphenyl β-D-glucopyranoside (4) is dissolved in a solvent such as acetonitrile, added with benzaldehyde dimethyl acetal and camphorsulfonic acid (acid catalyst), and agitated at 25°C-80°C for 2 hours or longer to give Compound 5. Subsequently, Compound 5, 2,3,4,6-tetra-O-acetyl-β-D-glucosypyranosyl 2,2,2-trichloroacetimidate (6) and 4Å molecular sieves are dissolved in a solvent such as dichloromethane, added with trimethylylsilyl trifluoromethanesulfonate at a low temperature (e.g. 0°C), and agitated at room temperature for 2 hours or longer to give Compound 7.

Compound 7 is dissolved in a solvent such as ethanol, added with P-toluenesulfonic acid at room temperature, agitated at 60°C-80°C for 2 hours or longer to complete the reaction, then neutralized with triethylamine, and concentrated under a reduced pressure. The resulting syrup is dissolved in a solvent such as pyridine, and added with acetic anhydride or the like to give Compound 8 with protected hydroxyl groups. Compound 8 is dissolved in acetonitrile and water, added with an oxidant such as cerium ammonium nitrate, and agitated for 5 minutes to 2 hours, thereby obtaining Compound 9.

### (C) Third step (Synthesis of compound represented by Formula (1))

The compound represented by Formula (1) can be synthesized, for example, by following Scheme 4 below using Compounds 3 and 9 obtained in Steps 1 and 2 above.

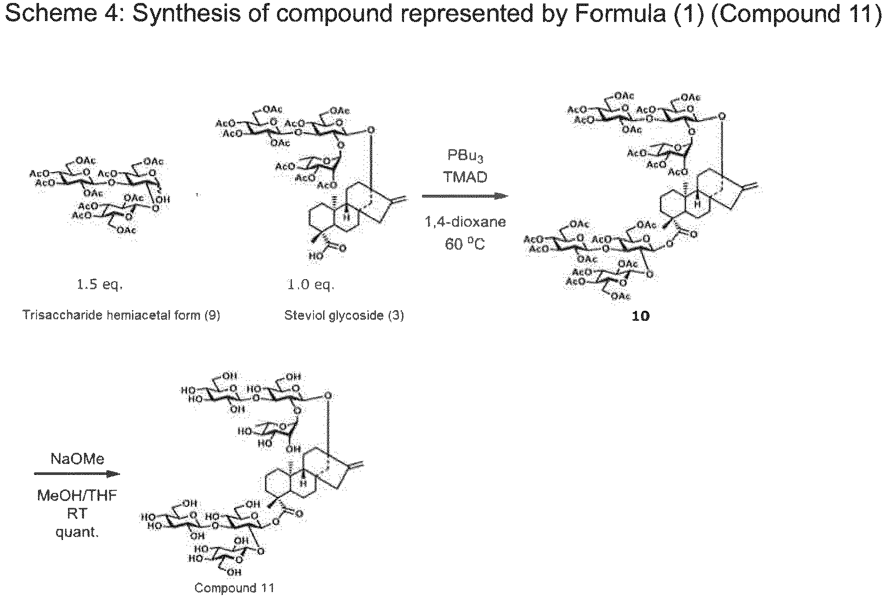

First, the trisaccharide hemiacetal form and the steviol glycoside obtained in Steps 1 and 2 are allowed to undergo the Mitsunobu reaction so that only Compound 10 in the β-form can selectively be obtained with very high yield (45% or more). Specifically, these compounds are dissolved in 1,4-dioxane, added with a phosphine reagent such as tributylphosphine or triphenylphosphine and an azo compound such as 1,1'-azobis (N,N'-dimethylformamide) (TMAD) at room temperature, and agitated at 50°C-80°C for 2 hours or longer to give only Compound 10 in the β-form. Finally, the protecting groups of Compound 10 are deprotected to give the compound represented by Formula (1) (Compound 11).

### (3) Biosynthesis

The steviol glycoside of the present invention can also be generated by transferring a polynucleotide coding for a predetermined protein into a host cell derived from a bacterium, a plant, an insect, a non-human mammal or the like, and using steviol, a steviol glycoside, UDP-glucose and/or UDP-rhamnose as a substrate. Steviol, a steviol glycoside, UDP-glucose or UDP-rhamnose as the substrate may be either provided or biosynthesized in the cell. While examples of the predetermined protein include stevia-derived UGT85C2 (the amino acid sequence represented by SEQ ID NO:2), UGT74G1 (the amino acid sequence represented by SEQ ID NO:4), UGT91D2 (the amino acid sequence represented by SEQ ID NO:6), UGT76G1 (the amino acid sequence represented by SEQ ID NO:8) and *Arabidopsis thaliana-derived* UDP-rhamnose synthase AtRHM2 (the amino acid sequence represented by SEQ ID NO: 10), it is not limited thereto as long as it has an equivalent activity.

The above-described protein is an enzyme derived from *Arabidopsis thaliana* or stevia, which is expected to be highly active in an environment outside plant cells such as *Arabidopsis thaliana* and stevia (for example, in an extracellular environment, or inside a host cell other than stevia). In this case, the polynucleotide coding for the above-described protein (for example, UGT85C2 gene is represented by SEQ ID NO:1, UGT74G1 gene is represented by SEQ ID NO:3, UGT91D2 gene is represented by SEQ ID NO:5, UGT76G1 gene is represented by SEQ ID NO:7 and AtRHM2 gene is represented by SEQ ID NO:9) is transferred into a host cell derived from a bacterium, a fungus, a plant, an insect or a non-human mammal so as to allow expression of the protein of the present invention, to which steviol, a steviol glycoside, UDP-glucose or UDP-rhamnose as the substrate is provided to generate the compound of the present invention. Alternatively, depending in the host, the above-described protein is expressed in the host cell, to which an appropriate substrate is provided to generate the compound of the present invention.

In one aspect of the present invention, a method for producing the novel steviol glycoside of the present invention is provided, where the method is characterized by use of a non-human transformant that has been introduced with at least one of polynucleotides (a) to (g) below.
(a) A polynucleotide containing the nucleotide sequence of SEQ ID NO:1, a polynucleotide containing a nucleotide sequence having 90% or higher identity with the nucleotide sequence of SEQ ID NO:1, or a polynucleotide coding for a protein that has 90% or higher identity with the amino acid sequence of SEQ ID NO:2 and that has an activity of adding glucose to the hydroxyl group at C-13 of the steviol glycoside.
(b) A polynucleotide containing the nucleotide sequence of SEQ ID NO:3, a polynucleotide containing a nucleotide sequence having 90% or higher identity with the nucleotide sequence of SEQ ID NO:3, or a polynucleotide coding for a protein that has 90% or higher identity with the amino acid sequence of SEQ ID NO:4 and that has an activity of adding glucose to the carboxylic acid at C-19 of the steviol glycoside.
(c) A polynucleotide containing the nucleotide sequence of SEQ ID NO:5, a polynucleotide containing a nucleotide sequence having 90% or higher identity with the nucleotide sequence of SEQ ID NO:5, or a polynucleotide coding for a protein that has 90% or higher identity with the amino acid sequence of SEQ ID NO:6 and that has an activity of adding rhamnose to glucose attached to C-13 of the steviol glycoside via a 1→2 linkage.
(d) A polynucleotide containing the nucleotide sequence of SEQ ID NO:7, a polynucleotide containing a nucleotide sequence having 90% or higher identity with the nucleotide sequence of SEQ ID NO:7, or a polynucleotide coding for a protein that has 90% or higher identity with the amino acid sequence of SEQ ID NO:8 and that has an activity of adding glucose to C-3 of glucose at C-13 of the steviol glycoside via a 1→3 linkage.
(e) A polynucleotide containing the nucleotide sequence of SEQ ID NO:5, a polynucleotide containing a nucleotide sequence having 90% or higher identity with the nucleotide sequence of SEQ ID NO:5, or a polynucleotide coding for a protein that has 90% or higher identity with the amino acid sequence of SEQ ID NO:6 and that has an activity of adding glucose to glucose at C-19 of the steviol glycoside via a 1→2 linkage.
(f) A polynucleotide containing the nucleotide sequence of SEQ ID NO:7, a polynucleotide containing a nucleotide sequence having 90% or higher identity with the nucleotide sequence of SEQ ID NO:7, or a polynucleotide coding for a protein that has 90% or higher identity with the amino acid sequence of SEQ ID NO:8 and that has an activity of adding glucose to glucose at C-19 of the steviol glycoside via a 1→3 linkage.
(g) A polynucleotide containing the nucleotide sequence of SEQ ID NO:9, a polynucleotide containing a nucleotide sequence having 90% or higher identity with the nucleotide sequence of SEQ ID NO:9, or a polynucleotide coding for a protein that has 90% or higher identity with the amino acid sequence of SEQ ID NO:10 and that has an activity of generating UDP-rhamnose from UDP-glucose.

In a preferable aspect of the present invention, polynucleotides independently having 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher sequence identity with the nucleotide sequences of the sequence numbers mentioned in (a) to (g) above can be used.

In another preferable aspect of the present invention, proteins that independently have an amino acid sequence having 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher sequence identity with the amino acid sequences of the sequence number mentioned in (a) to (g) above and that has the predetermined activity described in (a) to (g) above can be used.

Preferably, a polynucleotide coding for the above-described protein is introduced into a host while being inserted into an appropriate expression vector. The polynucleotides may individually be inserted into separate vectors.

An appropriate expression vector is generally made to contain:
(i) a promoter that allows transcription in the host cell;
(ii) a polynucleotide of the present invention linked to said promoter; and
(iii) an expression cassette that is involved in transcription termination and polyadenylation of RNA molecules and that contains, as a component thereof, a signal that functions in the host cell.

Examples of a method for preparing an expression vector include, but not particularly limited to, a method that uses a plasmid, a phage, a cosmid or the like, and DNA molecules having necessary components.

The type of the vector is not particularly limited, and any vector that allows expression in the host cell can suitably be selected. Specifically, a promoter sequence is suitably selected according to the type of the host cell to ensure expression of the polynucleotide of the present invention, and a vector obtained by integrating this promoter sequence and the polynucleotide of the present invention into a plasmid or the like is used as an expression vector.

The expression vector of the present invention includes expression controlling regions (for example, a promoter, a terminator and/or an origin of replication and the like) depending on the type of the host into which it is introduced. A promoter used in a bacterial expression vector may be a common promoter (for example, a trc promoter, a tac promoter, a lac promoter, etc.), a promoter used for a yeast may be, for example, a glyceraldehyde-3-phosphate dehydrogenase promoter, PH05 promoter, a GAL1/10 promoter or the like, and a promoter for filamentous fungi may be, for example, amylase, trpC or the like. Moreover, examples of a promoter for expressing the gene of interest in a plant cell include a cauliflower mosaic virus 35S RNA promoter, a rd29A gene promoter, a rbcS promoter, and a mac-1 promoter in which the enhancer sequence of the cauliflower mosaic virus 35S RNA promoter is provided at the 5' end of a promoter sequence of *Agrobacterium-derived* mannopine synthase. A promoter for an animal cell host may be a viral promoter (for example, a SV40 early promoter, a SV40 late promoter, etc.). Examples of a promoter that is inducibly activated in response to external stimuli include a mouse mammary tumor virus (MMTV) promoter, a tetracycline responsive promoter, a metallothionein promoter and a heat shock protein promoter.

Preferably, the expression vector contains at least one selectable marker. As such a marker, an auxotrophic marker (LEU2, URA3, HIS3, TRP1, ura5, niaD), a drug resistance marker (hygromycin, zeocin), a geneticin resistance gene (G418r), a copper resistance gene (CUP1) (Marin et al., Proc. Natl. Acad. Sci. USA, vol. 81, p. 337, 1984), a cerulenin resistance gene (fas2m, PDR4) (Junji Inokoshi et al., Journal of Japanese Biochemical Society, vol. 64, p. 660, 1992; Hussain et al., Gene, vol. 101, p. 149, 1991, respectively) or the like can be used.

As a method for transforming a host cell, a generally employed known method can be employed. For example, an electroporation method (Mackenxie, D. A. et al., Appl. Environ. Microbiol., vol. 66, p. 4655-4661, 2000), a particle delivery method (Japanese Unexamined Patent Application Publication No. 2005-287403), a spheroplast method (Proc. Natl. Acad. Sci. USA, vol. 75, p. 1929, 1978), a lithium acetate method (J. Bacteriology, vol. 153, p. 163, 1983), a method described in Methods in yeast genetics, 2000 Edition : A Cold Spring Harbor Laboratory Course Manual, or the like can be performed although the present invention is not limited thereto.

In addition, as to general molecular biological processes, see "Sambrook and Russell, Molecular Cloning: A Laboratory Manual Vol. 3, Cold Spring Harbor Laboratory Press 2001", "Methods in Yeast Genetics, A laboratory manual (Cold Spring Harbor Laboratory Press, Cold Spring Harbor, NY)" and the like.

A non-human transformant obtained as described above can be cultured so as to allow the non-human transformant to produce a steviol glycoside. Such a non-human transformant is preferably a yeast. Moreover, the non-human transformant is preferably cultured in a medium containing steviol. The accumulated steviol glycoside can be extracted/purified to obtain the steviol glycoside of the present invention.

### EXAMPLES

### Isolation of novel steviol glycoside

Extracts obtained from the leaves of four lines of novel stevia plants (Sample 1 (EM3-4), Sample 2 (EM2-27-8), Sample 3 (EM2-27-15) and Sample 4 (EM2-11)) developed at Suntory Global Innovation Center (SIC) were subjected to high performance liquid chromatography (HPLC)-mass spectrometry (MS) for the screening analysis of the contained steviol glycoside based on the molecular weights of a steviol glycoside that had a sugar chain formed of D-glucopyranosyl (glc), L-rhamnopyranosyl (rha) and xylopyranosyl (xyl). Here, Sample 1 is a high-Reb.C plant having a genome polymorphism of A in the wild type being altered to T at the 60th nucleotide of the nucleotide sequence represented by SEQ ID NO:11 in the genome of a test plant. A statistical analysis of the correlation between the phenotype having a high-RebC concentration and the polymorphism of SEQ ID NO:11 revealed that said polymorphism had a statistic correlation with the phenotype having a high-RebC concentration.

A process for preparing a test liquid was as follows: 10.0 mg each of lyophilized dried stevia leaves was weighed into a glass vial, to which 1.0 mL of water/methanol (1/1 vol/vol) was added as an extracting solvent, and then the resultant was subjected to ultrasonic irradiation in an ultrasonic cleaner (AS ONE, AS52GTU) at a set temperature of 25°C for 20 minutes, thereby obtaining a liquid extract of a steviol glycoside from the stevia leaves. The resultant was further 10-fold diluted with water/methanol and filtrated through a filter with a pore size of 0.45 µm (Nacalai tesque, Cosmonice filter S (solvent)) before being subjected to HPLC-MS.

For the HPLC part of HPLC-MS, Nexera LC-30AD (Shimadzu Corporation) was used as a liquid delivery unit LC pump, and SM-C18 (4.6 x 250 mm) (from Imtakt) as a separation column. Liquid delivery of the LC mobile phase was carried out by using 0.2% acetic acid-containing Milli-Q water as mobile phase A and methanol as mobile phase B, where the binary gradient was such that the concentration of the mobile phase B was constantly maintained at 10% for 0-5 minutes, the concentration of the mobile phase B was shifted from 10% to 70% in the next 15 minutes, then from 70% to 100% in the following 5 minutes, and finally ended by maintaining the concentration of the mobile phase B at 100% for 5 minutes. The flow rate of the mobile phase was 0.4 mL/min, and the stevia leaf liquid extract diluted and filtrated with a filter was injected for 5 µL.

For the MS part, triple quadrupole mass spectrometer LCMS-8030 (Shimadzu Corporation) equipped with an electrospray ionization (ESI) ion source was used. The mass spectrometry measurement was carried out in a selected ion monitoring (SIM) mode by selecting the negative ion measurement mode and the m/z values. The m/z values were selected by calculation based on the molecular weights of a steviol glycoside that had a sugar chain formed of D-glucopyranosyl (glc), L-rhamnopyranosyl (rha) and xylopyranosyl (xyl). Accordingly, m/z = 641.2 (glc (2)), 773.2 (glc (2), xyl (1)), 787.2 (glc (2), rha (1)), 803.2 (glc (3)), 935.3 (glc (3), xyl (1)), 949.3 (glc (3), rha (1)), 965.3 (glc (4)), 1095.4 (glc (3), rha (2)), 1097.4 (glc (4), xyl (1)), 1111.4 (glc (4), rha (1)), 1127.4 (glc (5)), 1257.5 (glc (4), rha (2)), 1259.5 (glc (5), xyl (1)), 1273.5 (glc (5), rha (1)), 1289.5 (glc (6)), 1435.6 (glc (6), rha (1)) were selected. Furthermore, an available high purity reagent, rebaudiosides A, B, D, F, M, N and O, stevioside, and dulcosides A and B were also measured under the same conditions so as to confirm the negative ion m/z values and the retention time of HPLC. The peak areas (arbitrary unit) of the mainly detected steviol glycosides are shown in Table 1.

### Table 1

**Table 1: Peak areas (arbitrary unit) observed by STM measurement in HPLC-MS**

| Compound name | Rebaudioside A | Rebaudioside C | Rebaudioside D | Rebaudioside M | Compound represented by Formula (1) | Rebaudioside N |
|---|---|---|---|---|---|---|
| Retention time (min) | 29.60 | 29.96 | 28.00 | 28.66 | 27.70 | 28.18 |
| Peak area (Sample 1) | 29,669,582 | 30,122,062 | 1,428,384 | 1,030,603 | 140,947 | 772,570 |
| | 46.97% | 47.69% | 2.26% | 1.63% | 0.22% | 1.22% |
| Peak area (Sample 2) | 23,762,676 | 24,201,473 | 2,253,735 | 1,029,837 | 97,388 | 1,211,504 |
| | 45.21% | 46.05% | 4.29% | 1.96% | 0.19% | 2.31 % |
| Peak area (Sample 3) | 15,386,726 | 5,872,656 | 3,585,775 | 3,296,579 | 89,988 | 896.549 |
| | 52.82% | 20.16% | 12.31% | 11.32% | 0.31% | 3.08% |
| Peak area (Sample 4) | 16,070,017 | 10,339,094 | 1,404,429 | 74,413 | 0 | 308,709 |
| | 56.99% | 36.67% | 4.98% | 0.26% | 0.00% | 1.09% |

Two peaks were observed in the selected ion chromatogram of the steviol glycoside (m/z 1273.5) in which the modified sugar chain contained five glucose moieties (glc) and one rhamnose moiety (rha). The selected ion chromatogram of Sample 1(EM3-4) at m/z of 1273.5 is shown in Figure 1.

Of the two peaks shown in Figure 2, the peak seen at the retention time (Rt) of 28.23 minutes matches the standard sample of rebaudioside N in terms of the mass value and the retention time. Meanwhile, no steviol glycoside has yet been reported to have a mass equivalent to that of rebaudioside N. Accordingly, the peak at Rt 27.73 minutes of the two peaks shown in Figure 2 was found to be an unknown substance. For Sample 4 whose rebaudioside C content was lower than the content of rebaudioside A and whose sugar chain elongation was shorter than other samples, the peak value at Rt 27.73 was lower than the detection limit.

### Structural analysis of novel steviol glycoside

According to the present invention, a structural analysis of the novel steviol glycoside detected in a cultivar with high rebaudioside C content was performed as follows.
(i) Structural deduction by a fragmentation analysis through high performance liquid chromatography (HPLC)-high resolution mass spectrometry (MS) and MS/MS, and three-stage ion fragmentation (MS³ fragmentation).
(ii) Chemical synthesis of the deduced steviol glycoside standard product via chemical reaction.
(iii) Structural confirmation by matching with the chemically synthesized standard product with respect to the retention time and the fragmented pattern from HPLC-high resolution MS and MS³ fragmentation

Hereinafter, each of Steps (i)-(iii) above will be described in detail.
(i) Structural deduction by a fragmentation analysis through high performance liquid chromatography (HPLC)-high resolution mass spectrometry (MS) and MS/MS, and three-stage ion fragmentation (MS³ fragmentation)

A process for preparing test liquids were as follows: 10.0 mg each of lyophilized dried stevia leaves was weighed into a glass vial, to which 1.0 mL of water/methanol (1/1 vol/vol) was added as an extracting solvent, and then the resultant was subjected to ultrasonic irradiation in an ultrasonic cleaner (AS ONE, AS52GTU) at a set temperature of 25°C for 20 minutes, thereby obtaining the liquid extract of a steviol glycoside from the stevia leaves. The resultant was further 10-fold diluted with water/methanol and filtrated through a filter with a pore size of 0.45 µm (Nacalai tesque, Cosmonice filter S (solvent)) before being subjected to HPLC-MS.

In an equipment configuration for high performance liquid chromatography-electrospray ionization-accurate mass spectrometry (HPLC-ESI-HRMS), equipment for HPLC was configured by using Prominence LC-20AD (Shimadzu Corporation) as a liquid delivery unit LC pump and SM-C18 (4.6 x 250 mm) (from Imtakt) as a separation column. The LC mobile phase was delivered using 0.2% acetic acid-containing Milli-Q water as mobile phase A and methanol as mobile phase B, where the binary gradient was such that the concentration of the mobile phase B was constantly maintained at 10% for 0-5 minutes, the concentration of the mobile phase B was shifted from 10% to 70% in the next 15 minutes, and further from 70% to 100% in the following 5 minutes. Finally, the concentration of the mobile phase B was maintained at 100% for 5 minutes to end. The flow rate of the mobile phase was 0.4 mL/min, and the stevia leaf liquid extract diluted and subsequently filtrated with a filter was injected for 5 µL. For the mass spectrometry part, Orbitrap Elite MS (from Thermo Fisher Scientific) equipped with an ESI ion source was used. The mass spectrometry measurement was carried out in a negative ion measurement mode at m/z in a range of 150-2000 with resolution set to 60,000. The MS/MS measurement was carried out by selecting the targeted m/z of 1273.5 and in a CID mode where fragmentation was induced by collision with an inert gas. Irradiation of energy required for fragmentation was performed at a standard collision energy unique to the apparatus, i.e. 35.

In order to study the fragmented pattern of the novel steviol glycoside, standard samples rebaudiosides A, D and M with known structures were subjected to MS/MS and MS³ fragmentation pattern analyses. As a result, MS/MS of the novel steviol glycoside gave data showing that the highest ion intensity appeared at the peak where the whole sugar chain attached to C-19 via an ester bond was released. This result shows the total molecular weight of the sugar chain attached to the carbon of C-19 via an ester bond.

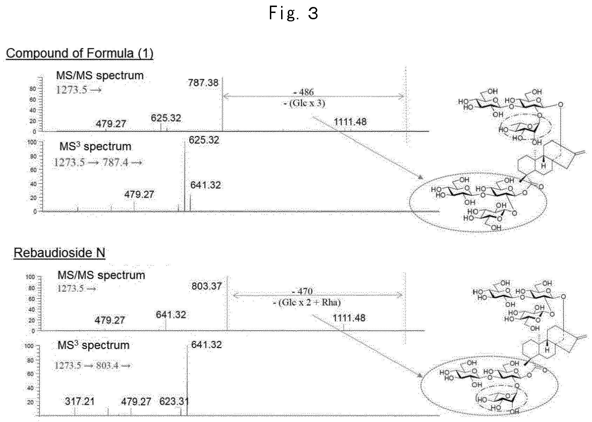

The MS/MS and MS³-fragmented mass spectra of rebaudioside N and the novel steviol glycoside are shown in Figure 3. When the MS/MS spectra of rebaudioside N and the novel steviol glycoside having the same MS value were compared, rebaudioside N had the main peak at m/z of 803.37 corresponding to release of two glc moieties and one rha moiety. On the other hand, in the MS/MS spectrum of the novel steviol glycoside, the main peak was detected at m/z of 787.38 corresponding to release of three Glc moieties. In order to acquire further structural information, a MS³ spectrum was acquired by fragmenting the main peak at m/z of 787.4 obtained by MS/MS. As a result, a spectrum having the same peak pattern as the MS³ spectrum of rebaudioside C (949.4→787.4→) was acquired. Accordingly, the sugar chain attached to C-13 was presumed to be the same as rebaudioside C. Considering that the stevia leaves of this sample also contained rebaudioside M, the sugar chain structure attached to C-19 of the novel steviol glycoside was presumed to be the same as the structure of rebaudioside M based on the potential of biosynthesis of the stevia leaves. The deduced structure is shown in Figure 3.

### (1) Outline of synthetic pathways

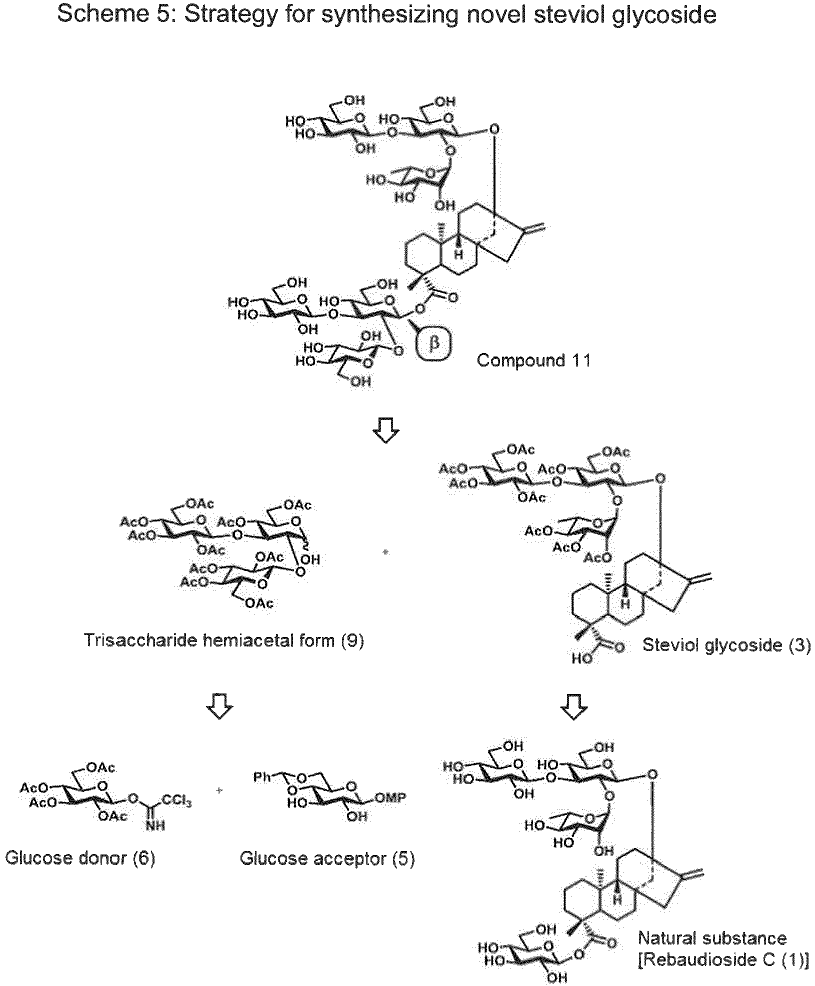

As can be appreciated from Scheme 5, for the synthesis of the novel steviol glycoside (11), the steviol glycoside (3) and the trisaccharide hemiacetal form (9) were condensed via the Mitsunobu reaction to obtain the backbone of the novel steviol glycoside (11). For synthesis of the steviol glycoside (3), a known natural substance, rebaudioside C (1), was purchased from Ark Pharm, the ester bond at C-19 of steviol was subjected to alkaline hydrolysis and then the hydroxyl groups of the sugar chain were protected with acetyl (Ac) groups to obtain the steviol glycoside (3). For synthesis of the trisaccharide hemiacetal form (9), a trisaccharide backbone was produced by condensation reaction between the appropriately protected glucose acceptor (5) and glucose donor (6), and the protecting group at the anomeric carbon of the reducing end was deprotected to give the trisaccharide hemiacetal form (9). The resulting steviol glycoside (3) and trisaccharide hemiacetal form (9) were subjected to condensation via the Mitsunobu reaction, where a reaction with good and complete β-selectivity of 47% (only the β-form) proceeded. The protecting groups of the resulting compound were deprotected, thereby obtaining the novel steviol glycoside (11).

Next, each of the synthesis steps will be described.

### (2) Synthesis of steviol glycoside

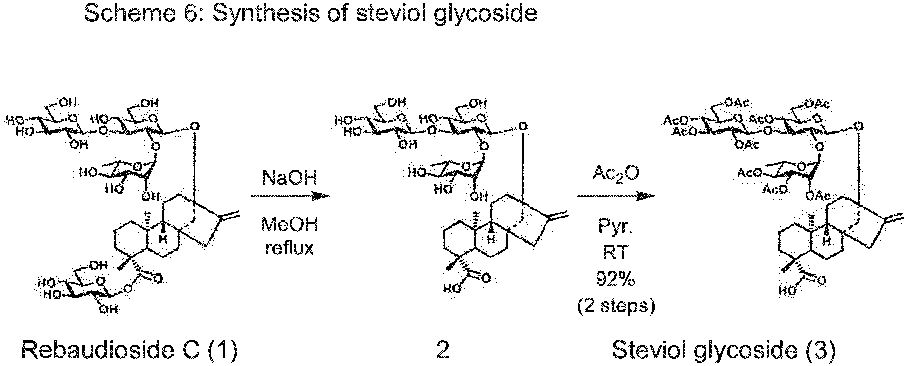

As can be appreciated from Scheme 6, for synthesis of the steviol glycoside (3), rebaudioside C (1) (1.0 g, 1.05 mmol) purchased from Ark Pharm was dissolved in methanol (10 mL) and water (10 mL), added with 4 mol/L of sodium hydroxide (2.6 mL, 10.5 mmol) at room temperature, and refluxed at 100°C for 20 hours. The completion of the reaction was confirmed by TLC (chloroform/methanol/water = 5/4/0.1, Rf value = 0.9) before the reaction solution was neutralized with cation exchange resin Dowex MAC-3 hydrogen form (SIGMA-ALDRICH) (pH 7). After the resin was removed by filtration, the resultant was concentrated under a reduced pressure. The resulting syrup was dried for 18 hours by using a vacuum pump to give Compound 2 (828 mg, quant.).

Compound 2 (828 mg, 1.05 mmol) was dissolved in pyridine (20 mL), added with acetic anhydride (5 mL) at room temperature and agitated for 48 hours at room temperature. After confirming the completion of the reaction by TLC (ethyl acetate/hexane = 2/1, Rf value = 0.5), a saturated sodium hydrogen carbonate solution (5 mL) was added, and the reaction solution was concentrated under a reduced pressure. The resulting syrup was subjected to silica gel column chromatography and an eluate (ethyl acetate/hexane = 2/1) was used to give Compound 3 (1.1 g, 92 %).

### [Compound 3]

¹H-NMR (CDCl₃, 400 MHz) δ 0.81 (m, 2H), 0.83-1.45 (complex, 19H), 1.39-1.91 (complex, 24H), 1.91-2.35 (s, 30H), 3.58 (m, 1H), 3.71-3.81 (complex, 4H), 3.95-4.12 (complex, 7H), 4.34-4.46 (complex, 3H), 4.56-4.66 (complex, 4H), 4.69-4.92 (complex, 7H), 5.05-5.14 (complex, 5H), 5.23-5.38 (complex, 6H), 5.45 (s, 1H); ¹³C-NMR (CDCl₃, 100 MHz) δ 15.9, 17.3, 19.1, 20.5, 20.7, 20.8, 20.9, 21.1, 21.5, 21.7, 29.1, 37.8, 38.0, 39.5, 40.7, 41.4, 42.2, 43.8, 48.4, 53.8, 56.8, 61.6, 63.0, 65.5, 66.8, 68.0, 68.6, 69.3, 69.6, 69.8, 70.5, 70.9, 71.6, 71.9, 72.4, 72.8, 73.9, 74.9, 81.3, 87.3, 96.6, 96.8, 99.2, 99.4, 125.4, 128.3, 129.1, 137.9, 151.9, 168.9, 169.2, 169.5, 169.6, 169.8, 170.1, 170.2, 170.3, 170.6, 170.9, 176.8, 183.4

### (3) Synthesis of trisaccharide hemiacetal form

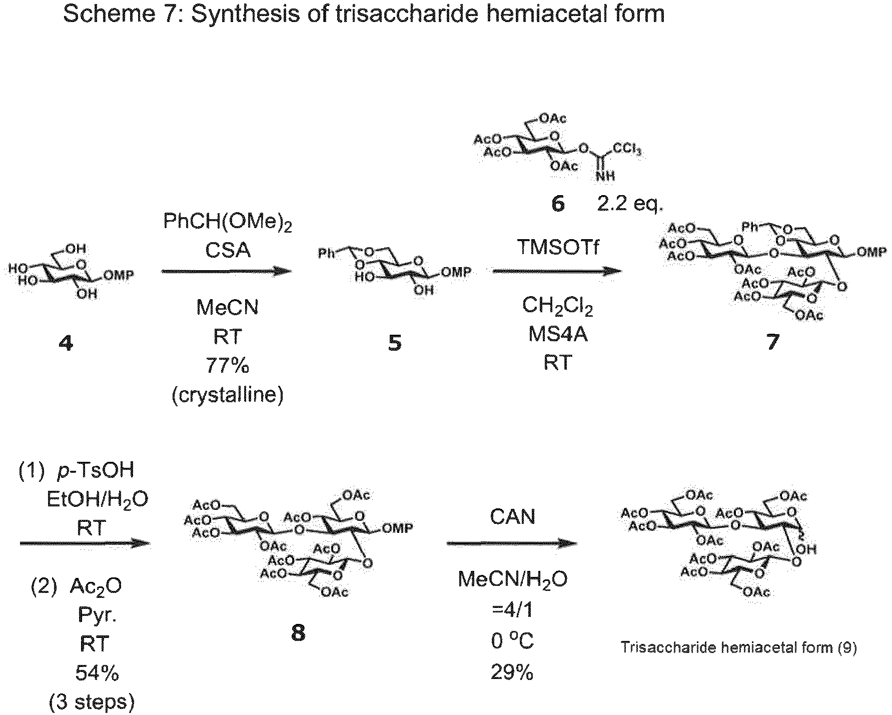

As can be appreciated from Scheme 7, for synthesis of the trisaccharide hemiacetal form (9), 4-methoxyphenyl β-D-glucopyranoside (4) (4.0 g, 13.9 mmol) purchased from Tokyo Chemical Industry was dissolved in acetonitrile (70 mL), added with benzaldehyde dimethyl acetal (3.1 mL, 20.9 mmol) and camphorsulfonic acid (323 mg, 1.39 mmol) at room temperature, and agitated at 50°C for 18 hours. After confirming the completion of the reaction by TLC (chloroform/methanol = 10/1, Rf value = 0.5), the resultant was neutralized with triethylamine (1 mL) (pH 8) and concentrated under a reduced pressure. The resulting residue was crystallized (ethanol) to give Compound 5 (4.0 g, 77%).

### [Compound 5]

¹H-NMR (CDCl₃, 400 MHz) δ 3.58 (m, 1H, H-5), 3.65 (t, 1H, H-4), 3.78 (m, 5H, H-2, H-6, OMe), 3.92 (t, 1H, H-3), 4.38 (dd, 1H, H-6'), 4.92 (d, J = 7.6 Hz, 1H, H-1), 5.57 (s, 1H, CHPh), 6.84 (dd, 4H, OMePh), 7.49 (m, 5H, Ph); ¹³C-NMR (CDCl₃, 100 MHz) δ 31.1, 55.8, 66.7, 68.8, 73.4, 74.6, 80.5, 102.2, 102.5, 114.8, 118.8, 126.4, 128.5, 129.5, 136.9, 150.9, 155.9

Compound 5 (1.0 g, 2.67 mmol), 3,4,6-tetra-O-acetyl-β-D-glucosypyranosyl 2,2,2-trichloroacetimidate (6) (2.9 g, 5.88 mmol) purchased from Tokyo Chemical Industry and 4Å molecular sieves (2.0 g) were dissolved in dichloromethane (171 mL), added with trimethylylsilyl trifluoromethanesulfonate (48 µL, 0.27 mmol) at 0°C, and agitated at room temperature for 1.5 hours. After confirming the completion of the reaction by TLC (toluene/ethyl acetate = 1/1, Rf value = 0.7), the resultant was neutralized with triethylamine (100 µL) (pH 8), 4Å molecular sieves was removed by filtration, and the resultant was concentrated under a reduced pressure. The resulting syrup was subjected to silica gel column chromatography and an eluate (toluene/ethyl acetate = 2/1) was used to give Compound 7.

Compound 7 (1.9 g, 1.84 mmol) was dissolved in ethanol (18 mL), added with P-toluenesulfonic acid (35 mg, 0.184 mmol) at room temperature, and agitated at 60°C for 5.5 hours. After confirming the completion of the reaction by TLC (ethyl acetate/hexane = 2/1, Rf value = 0.1), the resultant was neutralized with triethylamine (5.0 mL) (pH 8) and concentrated under a reduced pressure. The resulting syrup was dissolved in pyridine (18 mL), added with acetic anhydride (347 µL, 3.68 mmol) at room temperature, and agitated at room temperature for 18 hours. After confirming the completion of the reaction by TLC (ethyl acetate/hexane = 2/1, Rf value = 0.7), azeotropic distillation with toluene (30 mL) was repeated for three times, and the resultant was concentrated under a reduced pressure. The resulting syrup was subjected to silica gel column chromatography and an eluate (ethyl acetate/hexane = 1/1 → 2/1) was used to give Compound 8 (1.5 g, 54%, 3 steps).

### [Compound 8]

¹H-NMR (CDCl₃, 400 MHz) δ 1.94-2.17 (complex, 30H, OAc), 2.91 (m, 1H), 3.33 (m, 1H), 3.71 (m, 1H), 3.76 (m, 5H), 3.94 (t, 1H), 4.09-4.17 (complex, 5H), 4.31 (dd, 1H), 4.88 (m, 3H), 4.96-5.08 (complex, 4H), 5.18 (m, 2H), 5.26 (t, 1H), 6.84 (dd, OMePh); ¹³C-NMR (CDCl₃, 100 MHz) δ 20.6 x 2, 20.7 x 4, 20.8 x 2, 20.9, 21.1, 55.8, 60.3, 60.5, 61.8, 62.6, 67.6, 68.3, 71.4, 71.6, 71.9, 71.0, 72.1, 72.7, 73.1, 82.3, 98.5, 98.7 x 2, 114.9, 116.1, 150.1, 155.4, 168.9, 169.4 x 2, 169.5, 170.2, 170.3, 170.4, 170.5

Compound 8 (1.3 g, 1.26 mmol) was dissolved in acetonitrile (20 mL) and water (5.0 mL), added with cerium ammonium nitrate (1.4 g, 2.52 mmol) at 0°C and agitated at 0°C for 15 minutes. After confirming the completion of the reaction by TLC (ethyl acetate/hexane = 2/1, Rf value = 0.3), the resultant was diluted with ethyl acetate, and the organic layer was washed with water and a saturated aqueous sodium hydrogen carbonate solution and dried with magnesium sulfate. Magnesium sulfate was removed by filtration and the resultant was concentrated under a reduced pressure. The resulting syrup was subjected to silica gel column chromatography and an eluate (ethyl acetate/hexane = 2/1) was used to give Compound 9 (343 mg, 29%).

### [Compound 9]

¹H-NMR (CDCl₃, 400 MHz) δ 1.95-2.33 (complex, 55H, OAc), 3.61 (m, 6H), 3.73 (m, 1H), 3.91-4.31 (complex, 12H), 4.40 (m, 2H), 4.61 (d, J = 7.6 Hz, 1H), 4.65 (d, J = 7.6 Hz, 2H), 4.73 (d, J = 8.0 Hz, 1H), 4.82 (d, J = 8.0 Hz, 1H), 4.85-4.98 (complex, 4H), 5.01-5.21 (complex, 9H), 5.41 (d, J = 3.2 Hz, 1H); ¹³C-NMR (CDCl₃, 100 MHz) δ 20.8, 20.9 x 3, 21.1, 21.2, 21.5, 29.4, 29.8, 61.6, 61.7 x 2, 61.9, 62.4, 62.5, 67.3, 67.5, 67.9, 68.1 x 2, 68.2, 68.3, 71.6, 71.8, 71.9 x 2, 71.1, 72.2, 72.3, 72.8, 73.0, 74.8, 77.4, 78.3, 81.7, 82.8, 92.2, 95.6, 98.9, 99.5, 100.1, 101.5, 125.4, 128.3, 129.1, 137.9, 168.9, 169.4, 169.5 x 2, 169.9, 170.0, 170.1 x 2, 170.3 x 2

### (4) Synthesis of Compound 11

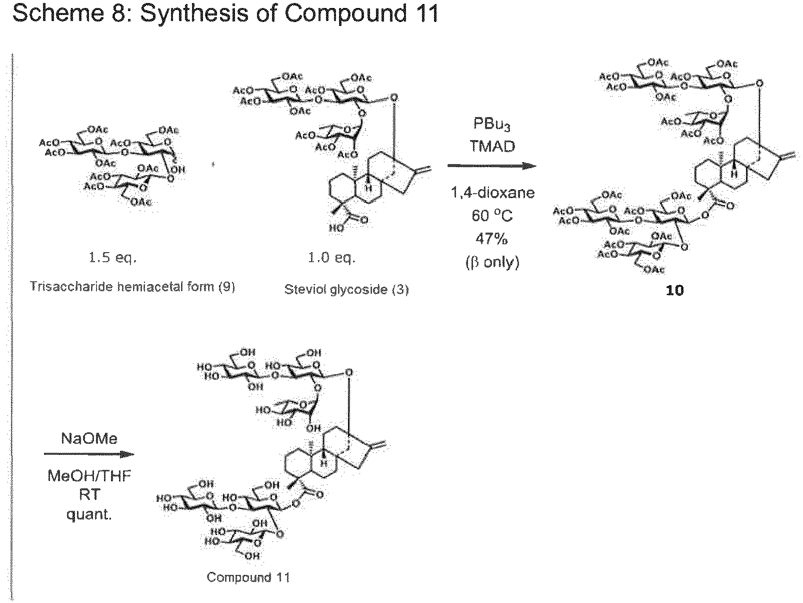

As can be appreciated from Scheme 8, for synthesis of Compound 11, Compound (9) (343 mg, 0.371 mmol) and Compound (3) (289 mg, 0.247 mmol) were dissolved in 1,4-dioxane (12 mL), added with tributylphosphine (185 µL, 0.741 mmol) and 1,1'-azobis (N,N'-dimethylformamide) (TMAD) (128 mg, 0.741 mmol) at room temperature, and agitated at 60°C for 18 hours. After confirming the completion of the reaction by TLC (toluene/ethyl acetate = 1/2, Rf value = 0.6), the resultant was diluted with ethyl acetate, and the organic layer was washed with water, a saturated aqueous sodium hydrogen carbonate solution and saturated saline, and dried with magnesium sulfate. Magnesium sulfate was removed by filtration and the resultant was concentrated under a reduced pressure. The resulting syrup was subjected to silica gel column chromatography and an eluate (toluene/ethyl acetate = 1/1) was used to give Compound 10 (240 mg, 47%).

### [Compound 10]

¹H-NMR (CDCl₃, 400 MHz) δ 0.50-1.05 (complex, 7H), 1.19 (d, 3H, H-6 of Rham), 1.23 (s, 3H), 1.35-2.30 (complex, 80H), 3.59 (m, 1H), 3.72 (m, 5H), 3.92-4.11 (complex, 10H), 4.21 (dd, 1H), 4.31 (dd, 1H), 4.42 (m, 3H), 4.60 (d, J = 7.6 Hz, 1H), 4.71-4.95 (complex, 9H), 5.07 (m, 6H), 5.19 (t, 1H), 5.29 (m, 4H), 5.59 (d, J = 7.6 Hz, 1H); ¹³C-NMR (CDCl₃, 100 MHz) δ 16.7, 17.4, 20.5, 20.7 x 3, 20.8, 20.9 x 4, 21.1 x 2, 21.6, 29.2, 39.5, 42.5, 44.2, 53.8, 57.4, 61.9, 66.7, 68.0, 68.3, 68.4, 68.5, 69.7, 71.1, 71.5, 71.8, 71.9, 72.0, 72.2, 72.3, 72.4, 72.9, 73.0, 75.0, 80.1, 86.8, 91.3, 96.4, 96.9, 99.2, 99.3, 99.5, 125.4, 128.3, 129.2, 152.8, 169.0, 169.1, 169.3, 169.5 x 2, 169.6, 170.1 x 2, 170.2 x 2, 170.5, 170.6, 170.9, 174.8

Compound (10) (220 mg, 0.106 mmol) was dissolved in methanol (2.0 mL) and THF (2.0 mL), added with sodium methoxide (0.5 M in MeOH) (0.2 mL, 0.106 mmol) at room temperature, and agitated at room temperature for 48 hours. After confirming the completion of the reaction by TLC (chloroform/methanol/water = 5/4/1, Rf value = 0.3), the resultant was concentrated under a reduced pressure. The resulting syrup was subjected to gel filtration column (GE Healthcare, Sephadex LH-20, ethanol) and lyophilized with water to give Compound 11 (135 mg, quant.).

### [Compound 11]

¹H-NMR (pyridine-d5, 800 MHz) δ 0.68 (m, 1H), 0.86 (m, 1H), 0.97 (m, 1H), 1.04 (m, 4H), 1.28 (m, 1H), 1.43 (m, 5H), 1.63 (s, 3H), 1.70 (s, 3H), 1.93-2.20 (complex, 8H), 2.40 (d, 1H), 2.84 (d, 1H), 3.64 (m, 1H), 3.82 (m, 1H), 3.94-4.15 (complex, 13H), 4.17-4.35 (complex, 14H), 4.45-4.59 (complex, 5H), 4.88 (m, 2H), 4.99 (d, J = 7.2 Hz,1H), 5.07 (s, 1H), 5.14 (d, J = 8.0 Hz, 1H), 5.32 (d, J = 8.0 Hz, 1H), 5.65 (s, 1H), 5.72 (d, J = 8.0 Hz, 1H), 6.20 (d, J = 8.0 Hz, 1H), 6.48 (s, 1H); ¹³C-NMR (pyridine-d5, 200 MHz) δ 17.0, 19.1, 20.2, 20.9, 22.3, 29.4, 37.7, 38.5, 39.9, 40.8, 41.9, 42.7, 43.4, 44.7, 48.4, 49.8, 54.1, 57.7, 61.9, 62.4, 62.5, 62.6, 63.6, 69.4, 69.8 x 2, 71.6, 71.7, 72.5, 72.8, 74.1, 75.2, 75.5, 76.0, 76.4, 77.5, 77.6, 78.5 x 2, 78.6 x 2, 78.8 x 2, 86.9, 88.5, 89.8, 93.5, 98.4, 101.9, 103.9, 104.4, 104.8, 105.2, 154.5, 176.1

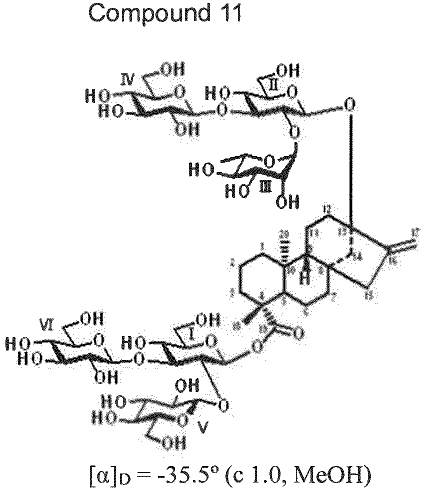

MALDI-TOF-MS m/z found [M + Na]⁺ 1297, C₅₆H₉₀O₃₂ calcd for [M + Na]⁺ 1297.

### (iii) Structural determination by matching with the chemically synthesized standard product with respect to the retention time and the fragmented pattern from HPLC-high resolution MS/MS and MS³ fragmentation

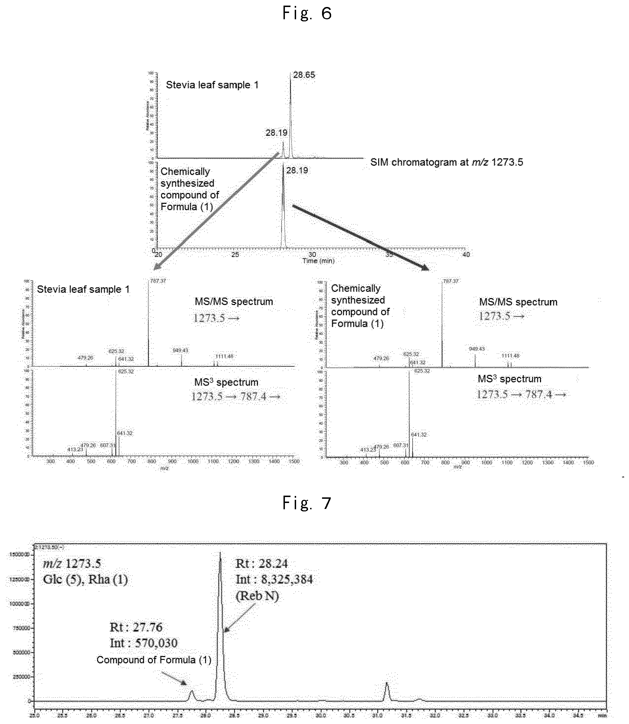

The chemically synthesized product (Compound 11) and stevia leaf liquid extracts were compared by HPLC-high resolution MS/MS and MS³-fragmentation under the same conditions as (i). As a result, the peaks of the chemically synthesized product and the stevia leaf liquid extract matched at the peak with the retention time of 28.19 minutes (Figure 6). From this result, the novel steviol glycoside obtained from the liquid extract of the plant was confirmed to have the same structure as Compound 11.

### Biosynthesis of novel steviol glycoside

A novel steviol glycoside was generated from steviol in yeast. First, a yeast capable of coexpressing four types of stevia-derived glycosylated enzyme genes UGT85C2, UGT91D2, UGT74G1 and UGT76G1 and *Arabidopsis thaliana-derived* UDP-rhamnose synthase gene AtRHM2 was bred.

Unless otherwise specified, the molecular biological processes employed in this example followed the methods described in Molecular Cloning (Sambrook et al., Cold Spring Harbour Laboratory Press, 2001).

In order to clone the four stevia-derived glycosylated enzyme genes, the following primer sets were synthesized to perform PCR using cDNA prepared from stevia leaves as a template.

Primer set for UGT85C2 gene amplification
CACC-NdeI-SrUGT85C2-Fw (NdeI-recognizing site underlined): 5'-CACCCATATGGATGCAATGGCTACAACTGAGAA-3' (SEQ ID NO:12)
BglII-SrUGT85C2-Rv (BglII-recognizing site underlined): 5'-AGATCTCTAGTTTCTTGCTAGCACGGTGATTT-3' (SEQ ID NO:13)

Primer set for UGT91D2 gene amplification
SrUGT91D2-pET15b-FW 5'-TGCCGCGCGGCAGCCATATGTACAACGTTACTTATCATC-3'(SEQ ID NO:35)
SrUGT91D2-pET15b-RV 5'-GTTAGCAGCCGGATCCTTAACTCTCATGATCGATGGCAA-3'(SEQ ID NO:36)

Primer set for UGT74G1 gene amplification
CACC-NdeI-SrUGT74G1-Fw (NdeI-recognizing site underlined): 5'-CACCCATATGGCGGAACAACAAAAGATCAAGAAAT-3'(SEQ ID NO:14)
BamHI-SrUGT74G1-Rv (BamHI-recognizing site underlined): 5'-GGATCCTTAAGCCTTAATTAGCTCACTTACAAATT-3' (SEQ ID NO:15)

Primer set for UGT76G1 gene amplification
CACC-NdeI-SrUGT76G1-Fw(NdeI-recognizing site underlined): 5'-CACCCATATGGAAAATAAAACGGAGACCA-3'(SEQ ID NO:16)
BamHI-SrUGT76G1-Rv (BamHI-recognizing site underlined): 5'-GGATCCTTACAACGATGAAATGTAAGAAACTA-3' (SEQ ID NO:17)
stevia leaf cDNA was obtained by extracting total RNA from stevia leaves using RNeasy Plant Mini kit (QIAGEN), and subjecting 0.5 µg of them to reverse transcription (RT) reaction using Random Oligo-dT primer.

The PCR reaction solution (50 µl) had the following composition: 1 µl of stevia leaf-derived cDNA, 1 x KOD plus buffer (TOYOBO), 0.2 mM dNTPs, 0.4 pmol/µl of each primer, 1 mM MgSO₄ and 1U heat resistant KOD plus polymerase. PCR reaction consisted of reaction at 95°C for 5 minutes, followed by amplification by a total of 30 cycles of reaction at 94°C for 0.5 minutes, 50°C for 0.5 minutes and 68°C for 2 minutes. Each PCR product was subjected to electrophoresis with 0.8% agarose gel and ethidium bromide staining, by which an amplification band of nearly 1.4 kb in size was obtained as presumed from each template DNA.

This PCR product was subcloned into pENTR-TOPO Directional vector (Invitrogen) according to a method recommended by the manufacturer. DNA Sequencer model 3100 (Applied Biosystems) was used for sequencing by a primer walking process with a synthesized oligonucleotide primer, thereby confirming that all of the UGT genes of interest, namely, UGT85C2, UGT91D2, UGT74G1 and UGT76G1 were cloned.

### Construction of yeast expression vector

The following primer sets were designed to integrate these UGT genes and *Arabidopsis thaliana-derived* UDP-rhamnose synthase gene AtRHM2 (J Biol Chem 2007, Oka et. al) into a yeast expression vector.

SrUGT85C2 set
Bgl2-UGT85C2-F (BglII-recognizing site underlined): 5'-ACAGATCTATGGATGCAATGGCTACAACTGAGA-3' (SEQ ID NO: 18)
Sal-UGT85C2-R (SalI-recognizing site underlined): 5'-TAGTCGACTAGTTTCTTGCTAGCACGGTGATTTC-3' (SEQ ID NO:19)

SrUGT91D2 set
NotI-UGT91DIL3-F (NotI-recognizing site underlined): 5'-AAGCGGCCGCATGTACAACGTTACTTATCATCAAAATTCAAA-3' (SEQ ID NO:20)
Pac-UGT91D1L3-R (PacI-recognizing site underlined): 5'-CGTTAATTAACTCTCATGATCGATGGCAACC-3' (SEQ ID NO:21)

SrUGT74G1 set
Not-UGT74G1-F (NotI-recognizing site underlined): 5'-AAGCGGCCGCATGGCGGAACAACAAAAGATCAAG-3' (SEQ ID NO:22)
Pac-UGT74G1-R (PacI-recognizing site underlined): 5'-CGTTAATTAAGCCTTAATTAGCTCACTTACAAATTCG-3' (SEQ ID NO:23)

SrUGT76G1 set
Bam-UGT76G1-F (BamHI-recognizing site underlined): 5'-AAGGATCCATGGAAAATAAAACGGAGACCACCG-3' (SEQ ID NO:24)
Sal-UGT76G1-R (SalI-recognizing site underlined): 5'-GCGTCGACTTACAACGATGAAATGTAAGAAACTAGAGACTCTAA-3' (SEQ ID NO:25)

AtRHM2 set
Bam-AtRHM2-F (BamHI-recognizing site underlined): 5'-GGATCCATGGATGATACTACGTATAAGCCAAAG-3' (SEQ ID NO:26)
Xho-AtRHM2-R (XhoI-recognizing site underlined): 5'-CTCGAGTTAGGTTCTCTTGTTTGGTTCAAAGA-3' (SEQ ID NO:27)

The combinations of templates and primers, namely, template UGT85C2 and SrUGT85C2 set, template UGT91D2 and SrUGT91D2 set, template UGT74G1 and SrUGT74G1 set, template UGT76G1 and SrUGT76G1 set, and template AtAHM2 and AtAHM2 set, were used for PCR amplification using heat resistant KOD DNA polymerase (TOYOBO) and introduction of the restriction enzyme sites at both ends of each ORF. The resulting DNA fragment was subcloned using Zero Blunt-TOPO PCR cloning kit (Invitrogen), and sequenced with DNA Sequencer model 3100 (Applied Biosystems) by a primer walking process with a synthesized oligonucleotide primer to confirm that each of the UGT genes of interest was cloned.

In order to express the above-described genes in yeasts by using pESC yeast expression system (Stratagene), the following expression vectors were constructed.

### (1) Construction of plasmid pESC-URA-UGT56

UGT85C2 was cleaved with restriction enzymes BglII and SalI, and linked to vector pESC-URA (Stratagene) that had been cleaved with restriction enzymes BamHI and SalI to give plasmid pESC-URA-UGT-5. This plasmid pESC-URA-UGT-5 was cleaved with restriction enzymes NotI and PacI while UGT91D2 was also cleaved with restriction enzymes NotI and PacI. The resultants were linked to give pESC-URA-UGT56.

### (2) Construction of plasmid pESC-HIS-UGT78

UGT76G1 was cleaved with restriction enzymes BamHI and SalI, and linked to vector pESC-HIS (Stratagene) that had been cleaved with the same restriction enzymes to give plasmid pESC-HIS-UGT-8. This plasmid pESC-HIS-UGT-8 was cleaved with restriction enzymes NotI and PacI while UGT74G1 was also cleaved with NotI and PacI. The resultants were linked to give pESC-HIS-UGT78.

### (3) Construction of plasmid pESC-TRP-AtRHM2

AtAHM2 was cleaved with restriction enzymes BamHI and XhoI while vector pESC-TRP (Stratagene) was cleaved with the same restriction enzymes. The resultants were linked to give plasmid pESC-TRP-AtAHM2.

### Transformation of yeast

Plasmids shown in Table 2 were introduced into *Saccharomyces cerevisiae* YPH499 strain (ura3-52 lys2-801^{amber}ade2-101^{ochre} trpl-Δ63 his3-Δ200 leu2-Δ1 a) as a host by lithium acetate technique. As transformed strains, those that survived in a SC-Trp-Ura-His agar medium (6.7 g of yeast nitrogen base without amino acids, 20 g of glucose, 1.3 g of amino acid powder mix-Trp-Ura-His and 20 g of Bacto agar per 1L) were selected.

**Table 2**

| Transformed strain | Plasmids introduced | Genes introduced |
|---|---|---|
| A-5678 | pESC-URA-UGT-56 | SrUGT85C2, SrUGT91D2 |
| | pESC-HIS-UGT-78 | SrUGT74G1, SrUGT76G1 |
| | pESC-TRP-AtAHM2 | AtAHM2 |

Here, the amino acid powder mix-Trp-Ura-His was prepared by mixing adenine sulfate (2.5 g), L-arginine hydrochloride (1.2 g), L-aspartic acid (6.0 g), L-glutamic acid (6.0 g), L-leucine (3.6 g), L-lysine (1.8 g), L-methionine (1.2 g), L-phenylalanine (3.0 g), L-serine (22.5 g), L-threonine (12 g), L-tyrosine (1.8 g) and L-valine (9.0 g).

### Induction and analysis of expression of transgene

The resulting transformed strain was cultured as follows.

First, for preliminary culture, each transformed strain was seeded in 10 ml of a SC-Trp-Ura-His liquid medium (SC-Trp-Ura-His agar medium without Bacto agar) and shake cultured at 30°C for a day. Subsequently, for main culture, 1 ml of the preliminary culture solution was seeded into 10 ml of SG-Trp-Ura-His liquid medium (6.7 g of yeast nitrogen base without amino acids, 20 g of galactose, and 1.3 g of amino acid powder mix-Trp-Ura-His per 1L) and shake cultured at 30°C for two days.

In order to confirm whether or not the introduced gene was expressed in the transformed strain, cells were harvested from the culture solution to purify total RNA with RNeasy Mini Kit.

With 1 µg of total RNA, cDNA was synthesized using SuperScript II reverse transcriptase (Thermo Fischer Scientific) and random hexamer as a primer.

In order to confirm expression of the transgene, the following primers were prepared.

For confirming expression of UGT85C2
UGT85C2-r1 : 5'-CAAGTCCCCAACCAAATTCCGT-3' (SEQ ID NO:28)

For confirming expression of UGT91D2
UGT91D1L3-r1 : 5'-CACGAACCCGTCTGGCAACTC-3' (SEQ ID NO:29)

For confirming expression of UGT74G1
UGT74G1-r1: 5'-CCCGTGTGATTTCTTCCACTTGTTC-3' (SEQ ID NO:30)

For confirming expression of UGT76G1
UGT76G1-r1: 5'-CAAGAACCCATCTGGCAACGG-3' (SEQ ID NO:31)

For confirming expression of AtAHM2
AtAHM2-rl 5'-GCTTTGTCACCAGAATCACCATT-3' (SEQ ID NO:32)

GAL10p region (promoter region)
PGAL10-f3: 5'-GATTATTAAACTTCTTTGCGTCCATCCA-3' (SEQ ID NO:33)

GAL1p region (promoter region)
PGAL1-f3: 5'-CCTCTATACTTTAACGTCAAGGAGAAAAAACC-3' (SEQ ID NO:34)

Expression of each transgene was confirmed by performing PCR by using ExTaq (Taraka Bio) with the following combination of primers and the previously synthesized cDNA as a template and subjecting the resulting product to agarose gel electrophoresis.
UGT85C2: UGT85C2-rl (SEQ ID NO:28) and PGAL1-f3 (SEQ ID NO:34)
UGT91D2 or UGT91D2L3: UGT91D1L3-r1 (SEQ ID NO:29) and PGAL10-f3 (SEQ ID NO:33)
UGT74G1: UGT74G1-r1 (SEQ ID NO:30) and PGAL1-f3 (SEQ ID NO:34)
UGT76G1: UGT76G1-r1 (SEQ ID NO:31) and PGAL10-f3 (SEQ ID NO:33)
AtAHM2: AtAHM2-rl (SEQ ID NO:32) and PGAL10-f3 (SEQ ID NO:33)

Accordingly, expression of the transgene in the transformed strain was confirmed.

### Production of novel steviol glycoside

Culturing was performed under the same conditions as described above except that 0.5 µg or 2 µg of steviol (ChromaDex Inc.) was added to the liquid medium for the main culture per 1 ml of the medium. After culturing, the culture solution was separated into supernatant and cells by centrifugation. The culture supernatant was washed with acetonitrile, then subjected to a water-equilibrated Sep-Pak C18 column, washed with 20% acetonitrile, eluted with 80% acetonitrile, dried to solidify, and then dissolved in a small amount of 80% acetonitrile to prepare a glycoside sample. This glycoside sample was subjected to the following analysis.

### Analysis by LC-MS

An analysis by LC-MS was carried out as described in the example under "Isolation of novel steviol glycoside".

The result is shown in Figure 7. Generation of the novel steviol glycoside in A-5678 strain was confirmed. This result corresponds to that for the steviol glycoside resulting from the above-described chemical synthesis.

### Evaluation of sweetness level of novel steviol glycoside

In order to evaluate the sweetness level of the novel steviol glycoside, samples were prepared by adding sucrose to pure water to give Brix of 0.5 to 3 in 0.5 increments. A sample was prepared by adding the novel steviol glycoside to pure water to 415 ppm.

Evaluation was conducted by selecting the sucrose-added sample having sweetness intensity equivalent to that of the sample added with the novel steviol glycoside, where sensory evaluation was conducted by panelists trained about sensory attributes of sweeteners (5 members). As a result, the sample prepared by adding the novel glycoside was found to have sweetness equivalent to that of the sucrose-added sample with Brix of 1. Therefore, the novel steviol glycoside of the invention was found to have a sweetness level of 24 with respect to sucrose.

### Sensory evaluation of novel steviol glycoside

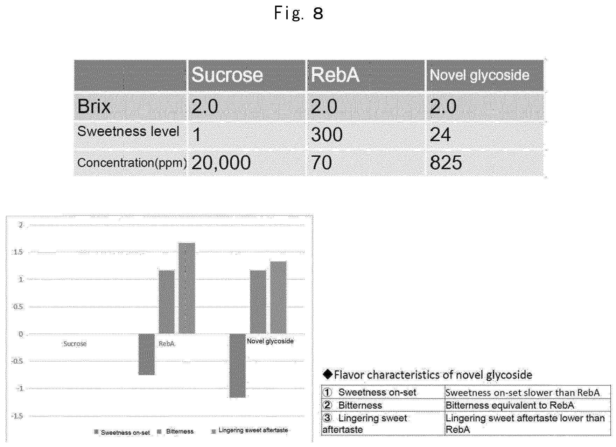

In order to evaluate the taste quality of various steviol glycosides, Reb.A and the novel steviol glycoside were added to pure water at amounts indicated in Figure 8 to prepare beverage samples. All of the beverage samples were adjusted to have final Brix of 2 in terms of sucrose, provided that the sweetness levels were RebA: 300 and novel glycoside: 24.

The resulting beverage samples were subjected to sensory evaluation for rating attributes, namely, sweetness on-set, bitterness and lingering sweet aftertaste. Panelists trained about sensory attributes of sweeteners (5 members) evaluated based on the following evaluation criteria. Very weak (-3), weak (-2), slightly weak (-1), normal (0), slightly strong (+1), strong (+2) and very strong (+3).

As a result of the sensory evaluation, the novel steviol glycoside was found to have equal bitterness and shorter lingering sweet aftertaste as compared to the conventional sweetener Reb.A.

### Evaluation of flavor controlling agent containing novel steviol glycoside for improving lingering aftertaste

### (1) Measurement of sweetness level of sweetener targeted for improvement of lingering aftertaste

Prior to evaluation of the flavor controlling agent, the sweetness level of the sweetener targeted for improvement of lingering aftertaste was measured. Reb.A (purity 100%) and Reb.D (purity 97%) were used as the sweeteners. Reb.A and Reb.D were dissolved in water in amounts indicated in the table below to prepare aqueous solutions. In the meantime, standard aqueous solutions having Brix of 5-7 were prepared using sucrose (sugar), for which panelists trained about sensory attributes of sweeteners (6 members) evaluated as to which standard aqueous solution had the corresponding sweetness to that of the aqueous Reb.A solution and the aqueous Reb.D solution. The results are shown in the table below.

**Table 3**

| | Aqueous Reb. A solution | Aqueous Reb. D solution |
|---|---|---|
| Concentration of sweetener in aqueous solution | 23.3 mg/100 ml | 28 mg/100 ml |
| Brix of corresponding standard aqueous solution (average evaluation value by 5 panelists) | 5.53 | 5.96 |
| Sweetness fold | 237 | 213 |

From the above results, evaluations in the following test were conducted provided that the sweetness fold of Reb.A was 237-fold and the sweetness fold of Reb.D was 213-fold. Here, the sweetness fold of the novel steviol glycoside used for evaluation was 24-fold as described above.

### (2) Evaluation of effect of improving lingering aftertaste of Reb.A

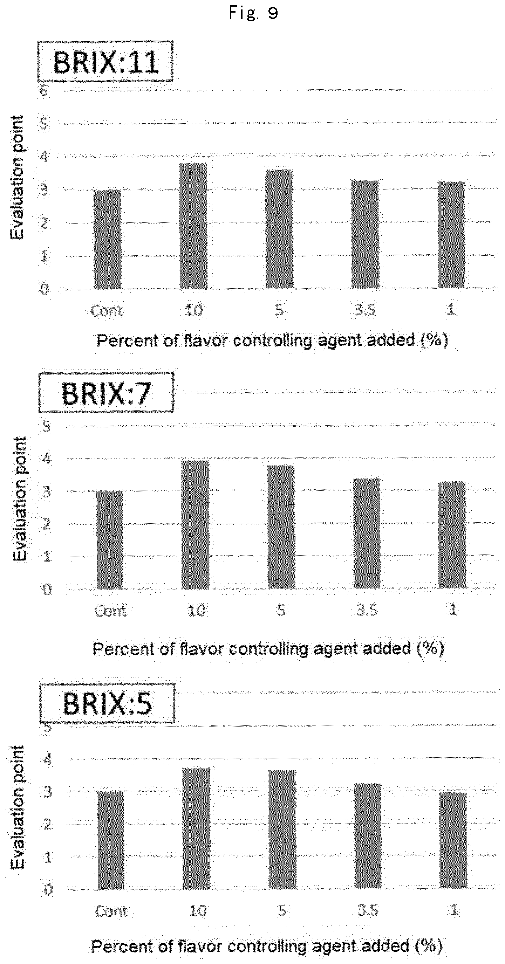

Three-level aqueous solutions with Brix of 5, 7 and 11 were used to evaluate the effect of the flavor controlling agent of the present invention to improve lingering aftertaste of Reb.A. First, three-level aqueous solutions with Brix of 5, 7 and 11 were generated provided that the sweetness level of Reb.A was 237-fold. The added amounts of the flavor controlling agent made of the novel steviol glycoside of the present invention were 1, 3.5, 5 and 10 mass% in proportion based on the mass of added Reb.A. A control sample (Cont) was added with Reb.A only and was not added with the flavor controlling agent of the present invention. Panelists trained about sensory attributes of sweeteners (7 members) conducted a numerical evaluation in which the maximum improvement of lingering aftertaste was set to 6 points while Cont was set to 3 points. The more the lingering aftertaste was improved, the higher the point was. The average evaluation points are shown in the graphs in Figure 9.

### (3) Evaluation of effect of improving lingering aftertaste of Reb.D

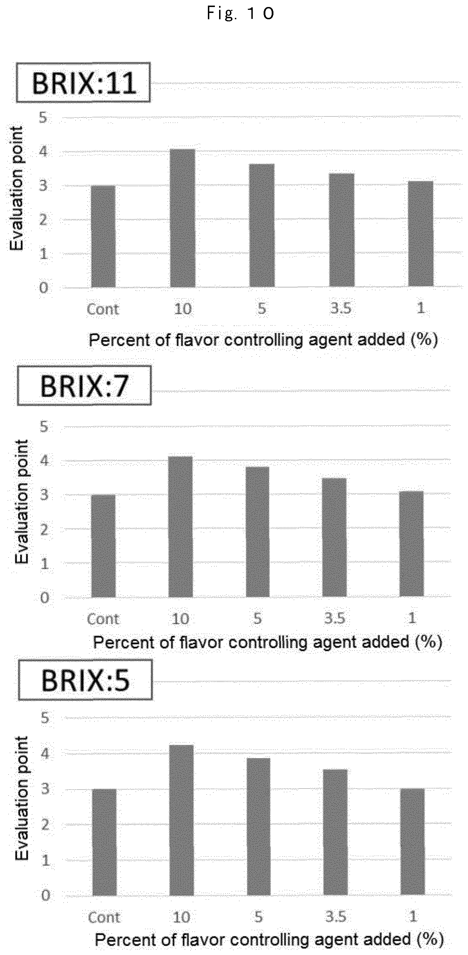

Three-level aqueous solutions with Brix of 5, 7 and 11 were used to evaluate the effect of the flavor controlling agent of the present invention to improve lingering aftertaste of Reb.D. First, three-level aqueous solutions with Brix of 5, 7 and 11 were generated provided that the sweetness level of Reb.D was 213-fold. The added amounts of the flavor controlling agent made of the novel steviol glycoside of the present invention were 1, 3.5, 5 and 10 mass% in proportion based on the mass of added Reb.D. A control sample (Cont) was added with Reb.D only and was not added with the flavor controlling agent of the present invention. Panelists trained about sensory attributes of sweeteners (7 members) conducted a numerical evaluation in which the maximum improvement of lingering aftertaste was set to 6 points while Cont was set to 3 points. The more the lingering aftertaste was improved, the higher the point was. The average evaluation points are shown in the graphs in Figure 10.

### Evaluation of flavor controlling agent containing novel steviol glycoside for enhancing sweetness

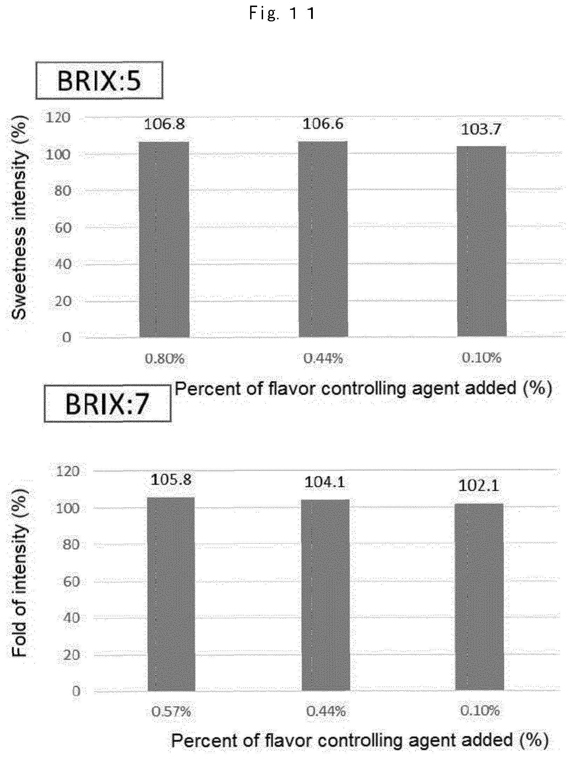

Two-level aqueous solutions with Brix of 5 and 7 were used to evaluate the effect of the flavor controlling agent of the present invention to enhance sweetness with respect to sugar (sucrose). First, two-level aqueous solutions with Brix of 5 and 7 were generated using sugar. The added amounts of the flavor controlling agent made of the novel steviol glycoside of the present invention were 0.10, 0.44 and 0.80 mass% in proportion in terms of the amount of the sugar added for Brix of 5, and 0.10, 0.44 and 0.57 mass% in proportion for Brix of 7. Panelists trained about sensory attributes of sweeteners (7 members) evaluated based on the rate of the sweetness intensity. The results are shown in graphs in Figure 11. The vertical axes (sweetness intensity) of the graphs represent the rates of sweetness intensity actually sensed by the panelists with respect to the total sweetness level of the sugar and the flavor controlling agent (calculated provided that the sweetness level of the flavor controlling agent had a sweetness fold of 24). For all samples, a sweetness enhancement effect of about 2-7% was observed.

## Claims

1. A compound represented by Formula (1): 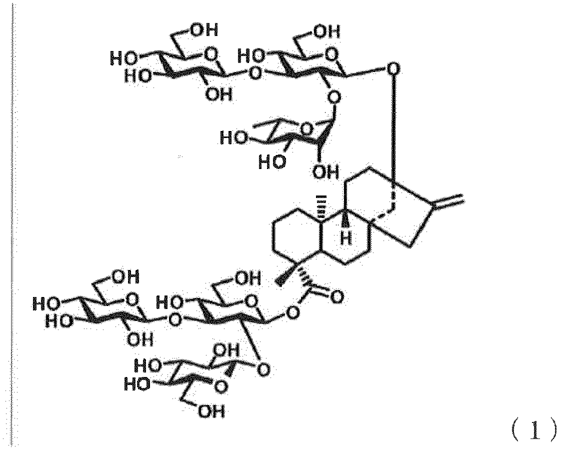 or a derivative, salt or hydrate thereof.

2. Compound according to Claim 1, or derivative, salt or hydrate thereof, wherein the compound or derivative, salt or hydrate thereof is a plant-derived product, a chemically synthesized product or a biosynthetic product.

3. A sweetener composition comprising a compound according to Claim 1 or 2, or a derivative, salt or hydrate thereof.

4. Sweetener composition according to Claim 3, further comprising one or more types of steviol glycoside selected from the group consisting of rebaudioside A, rebaudioside B, rebaudioside C, rebaudioside D, rebaudioside E, rebaudioside F, rebaudioside I, rebaudioside J, rebaudioside K, rebaudioside N, rebaudioside M, rebaudioside O, rebaudioside Q, rebaudioside R, dulcoside A, rubusoside, steviol, steviol monoside, steviol bioside and stevioside.

5. A food or beverage comprising a compound according to Claim 1 or 2, or a derivative, salt or hydrate thereof.

6. Food or beverage according to Claim 5, which is a beverage.

7. A plant comprising a compound according to Claim 1, or a derivative, salt or hydrate thereof.

8. An extract of a plant according to Claim 7.

9. A food or beverage comprising a plant according to Claim 7 or an extract of a plant according to Claim 8.

10. Food or beverage according to Claim 9, which is a beverage.

11. A method for producing a compound of Claim 1, comprising the steps of:
(A) synthesizing a compound represented by Formula (3) below: 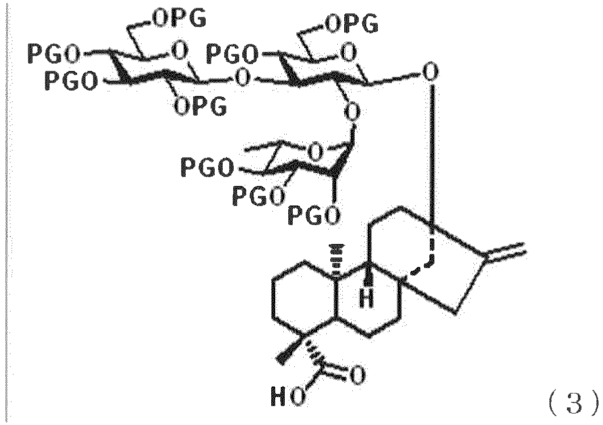 (wherein, PGs each independently represents a protecting group) from rebaudioside C represented by Formula (2) below: 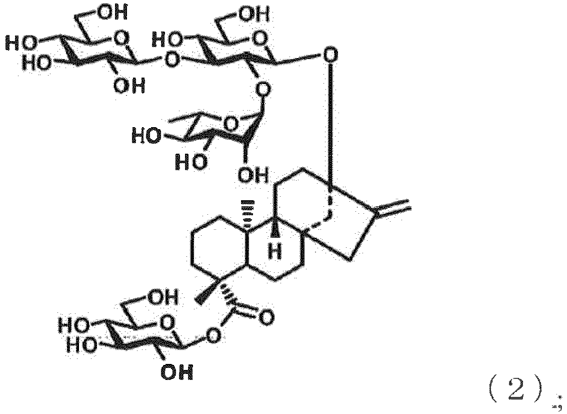 and
(B) synthesizing a compound represented by Formula (4) below: 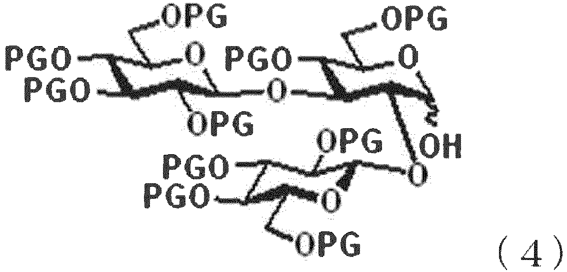 (wherein, PGs each independently represents a protecting group) from a glucopyranoside derivative.

12. A method according to Claim 11, comprising a step of allowing reaction between the compound represented by Formula (3) above and the compound represented by Formula (4) above in the presence of a phosphine reagent and an azo compound to obtain a compound represented by Formula (5) below: 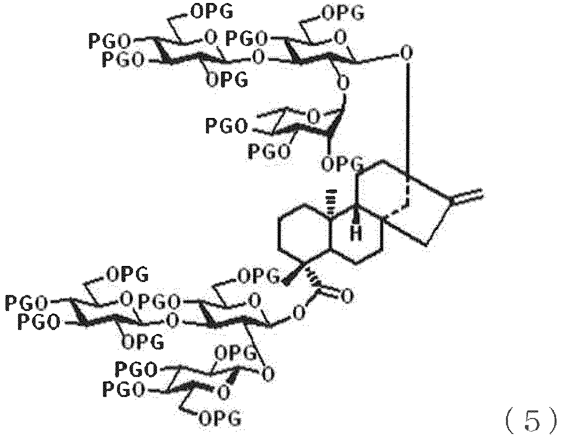 (wherein, PGs each independently represents a protecting group).

13. A method according to Claim 12, wherein the yield of the step of obtaining the compound represented by Formula (5) above is 40% or more.

14. Use of a compound according to Claim 1 or 2, or a derivative, salt or hydrate thereof, as a sweetener.

15. A method for producing a compound according to Claim 1, the method **characterized by** use of a non-human transformant that has been introduced with at least one of polynucleotides (a) to (g) below:
(a) a polynucleotide containing the nucleotide sequence of SEQ ID NO:1, a polynucleotide containing a nucleotide sequence having 90% or higher identity with the nucleotide sequence of SEQ ID NO:1, or a polynucleotide coding for a protein that has 90% or higher identity with the amino acid sequence of SEQ ID NO:2 and that has an activity of adding glucose to the hydroxyl group at C-13 of the steviol glycoside;
(b) a polynucleotide containing the nucleotide sequence of SEQ ID NO:3, a polynucleotide containing a nucleotide sequence having 90% or higher identity with the nucleotide sequence of SEQ ID NO:3, or a polynucleotide coding for a protein that has 90% or higher identity with the amino acid sequence of SEQ ID NO:4 and that has an activity of adding glucose to the carboxylic acid at C-19 of the steviol glycoside;
(c) a polynucleotide containing the nucleotide sequence of SEQ ID NO:5, a polynucleotide containing a nucleotide sequence having 90% or higher identity with the nucleotide sequence of SEQ ID NO:5, or a polynucleotide coding for a protein that has 90% or higher identity with the amino acid sequence of SEQ ID NO:6 and that has an activity of adding rhamnose to glucose attached to C-13 of the steviol glycoside via a 1→2 linkage;
(d) a polynucleotide containing the nucleotide sequence of SEQ ID NO:7, a polynucleotide containing a nucleotide sequence having 90% or higher identity with the nucleotide sequence of SEQ ID NO:7, or a polynucleotide coding for a protein that has 90% or higher identity with the amino acid sequence of SEQ ID NO:8 and that has an activity of adding glucose to C-3 of glucose at C-13 of the steviol glycoside via a 1→3 linkage;
(e) a polynucleotide containing the nucleotide sequence of SEQ ID NO:5, a polynucleotide containing a nucleotide sequence having 90% or higher identity with the nucleotide sequence of SEQ ID NO:5, or a polynucleotide coding for a protein that has 90% or higher identity with the amino acid sequence of SEQ ID NO:6 and that has an activity of adding glucose to glucose at C-19 of the steviol glycoside via a 1→2 linkage;
(f) a polynucleotide containing the nucleotide sequence of SEQ ID NO:7, a polynucleotide containing a nucleotide sequence having 90% or higher identity with the nucleotide sequence of SEQ ID NO:7, or a polynucleotide coding for a protein that has 90% or higher identity with the amino acid sequence of SEQ ID NO:8 and that has an activity of adding glucose to glucose at C-19 of the steviol glycoside via a 1→3 linkage; and
(g) a polynucleotide containing the nucleotide sequence of SEQ ID NO:9, a polynucleotide containing a nucleotide sequence having 90% or higher identity with the nucleotide sequence of SEQ ID NO:9, or a polynucleotide coding for a protein that has 90% or higher identity with the amino acid sequence of SEQ ID NO:10 and that has an activity of generating UDP-rhamnose from UDP-glucose.

16. A method according to Claim 15, wherein the non-human transformant is a yeast.

17. A method according to Claim 15 or 16, wherein the non-human transformant is cultured in a medium containing steviol.

18. A flavor controlling agent comprising a compound, or a derivative, salt or hydrate thereof, according to Claim 1.
